# EUROPEAN PATENT APPLICATION

(11) **EP 4 545 545 A1**
(43) Date of publication of application: **30.04.2025**
(21) Application number: 23834490.7
(22) Date of filing: 04.05.2023
(51) Int. Cl.: C07K 1/107, C07K 1/06

(54) **VISIBLE LIGHT-MEDIATED CYSTEINE-BASED POLYPEPTIDE AND PROTEIN CHEMICAL MODIFICATION METHOD**

(30) Priority: 04.07.2022 CN 202210780183; 21.03.2023 CN 202310280695
(71) Applicant: Shanghai Jiao Tong University, Shanghai 200240 (CN)
(72) Inventor: SUN, Zhankui, Shanghai 200240 (CN); ZHOU, Junliang, Shanghai 200240 (CN); LIU, Yunqi, Shanghai 200240 (CN)
(74) Representative: Bayramoglu et al.
(86) International application number: PCT/CN2023/092005
(87) International publication number: WO 2024/007714

(57) **Abstract**

The present disclosure discloses a method for visible light-mediated chemical modification of a polypeptide and protein based on cysteine, including the following steps: allowing a cysteine residue on a cysteine-containing oligopeptide, polypeptide, or protein to undergo a reaction with an activating reagent to generate a free radical precursor *in situ*; and subjecting the free radical precursor to thiol removal under light induction, and allowing the generated free radical intermediate to undergo an addition reaction with an olefin or an alkyne, such that sulfhydryl removal and carbon-carbon bond construction are achieved to produce a chemical modification product of the polypeptide and protein. The design strategy of the present disclosure cleverly avoids the influence on the chiral center, and thus the chirality of the amino acid residue can be retained. The whole design is conducive to well avoiding the occurrence of side reactions, and thus the efficiency of the entire conversion is very high. The reactions can be conducted mildly under physiological conditions, and can be compatible with various types of amino acid residues and olefins or alkynes. Therefore, the method has advantages such as high efficiency, wide application scope, and maintenance of amino acid residue configurations.

## Description

### TECHNICAL FIELD

The present disclosure belongs to the field of chemical marking for polypeptides and proteins, and relates to a method for visible light-mediated chemical modification of a polypeptide and protein based on cysteine. Specifically, the present disclosure relates to a method for visible light-mediated chemical modification of a polypeptide and protein with cysteine as a reaction site. This method can mildly achieve the chemical modification of a cysteine residue under the mediation of a photocatalyst to construct a carbon-carbon bond. This method can be used in fields such as chemical transformation for proteins, structural modification for enzymes, development of antibody-drug conjugates (ADCs), and development of degrader-antibody conjugates.

### BACKGROUND TECHNOLOGY

Posttranslational modifications for proteins play an important role in the regulation of structures and functions of proteins. For example, protein phosphorylation serves as a switch for many biological functions, protein glycosylation controls the physical and biological properties of proteins, and protein ubiquitination plays a very important role in the localization, metabolism, functions, regulation, and degradation of proteins. Most of the posttranslational modification processes for proteins that occur in the nature are based on heteroatoms on amino acids, such as sulfhydryl on cysteine, amino on lysine, and hydroxyl on serine and threonine, and are characterized by the formation of carbon-heteroatom bonds. Most of the current chemical modification methods developed for proteins are also based on these heteroatoms. In the nature, carbon-carbon bonds (usually carbon-carbon single bonds) serve as basic backbones of amino acids. Carbon-carbon bonds have ultra-high stability, but carbon-heteroatom bonds have poor stability. For example, in most of the existing ADCs, a C-S bond is formed through a reaction between cysteine and a maleimide fragment to connect an antibody and a small molecule fragment. However, in the blood circulation, such a connection mode is prone to a reverse Micheal addition reaction to generate a small molecule fragment, and the small molecule fragment will react with sulfhydryl-containing fragments such as albumin and glutathione in the blood, resulting in toxicity (as shown in the figure below, B. Q. Shen. et al. Conjugation site modulates the in vivo stability and therapeutic activity of antibody-drug conjugates. Nat. Biotechnol 30, 184-189 (2012)). As the first ADC in China, RC48 of RemeGen is highly toxic mainly because RC48 adopts a linker between cysteine and maleimide. This C-S bond linker can undergo a reverse Michael reaction in the plasma, which induces the off-target to cause toxicity.

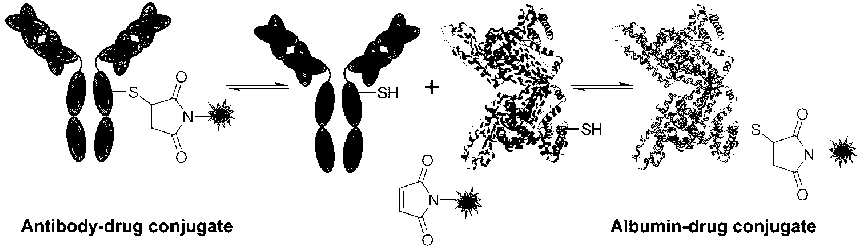

In the technology disclosed in the present disclosure, with a cysteine residue as a reaction site, a carbon-carbon bond can be constructed, which well solves the above problem. Thus, the technology disclosed in the present disclosure is expected to be used in fields such as the development of ADCs, the development of degrader-antibody conjugates, and structural modification for proteins. The present disclosure confirms through plasma stability experiments that the synthesized carbon-carbon bond product exhibits much better plasma stability than the corresponding carbon-sulfur bond compound.

In 2016, B. G. Davis and Hee-Sung Park, et al. published the respective protein modification methods on the Science journal successively (B. G. Davis et al. Posttranslational mutagenesis: A chemical strategy for exploring protein side-chain diversity. Science 354, 1465-1481 (2016). A. Yang, S. Ha, J. Ahn, R. Kim, S. Kim, Y. Lee, J. Kim, D. Soll, H. Y. Lee, H. S. Park. A chemical biology route to site-specific authentic protein modifications. Science 354, 623-626 (2016). B. G. Davis. et al. Light-driven posttranslational installation of reactive protein side chains. Nature 585, 530-537 (2020).). In these protein modification methods, the construction of carbon-carbon bonds on side chains of proteins are achieved based on free radical reactions. In addition, a variety of unnatural amino acids can be introduced to allow a variety of posttranslational modifications including methylation, phosphorylation, and glycosylation. However, products of the above two methods have two different configurations, which limits the application of these two methods. Subsequently, other chemical modification methods for proteins have been developed, but each have limitations. For example, the method developed by MacMillan is merely suitable for the modification at a C-terminus of a protein (W. C. MacMillan et al. Decarboxylative alkylation for site-selective bioconjugation of native proteins via oxidation potentials. Nat. Chem. 10, 205-211 (2018).). The method developed by Zhi-Cai Shi and Nicholas J. Mitchell has a low yield (Y. Yu, L. Zhang, A. V. Buevich, G. Li, H. Tang, P. Vachal, S. L. Colletti, Z. C. Shi. Chemoselective peptide modification via photocatalytic tryptophan β-position conjugation. J. Am. Chem. Soc. 140, 6797-6800 (2018). R. C. Griffiths, F. R. Smith, J. E. Long, D. Scott, H. E. L. Williams, N. J. Oldham, R. Layfield, N. J. Mitchell. Site-selective installation of Ne-modified sidechains into peptide and protein scaffolds via visible-light-mediated desulfurative C-C Bond Formation. Angew. Chem. Int. Ed. 61, e202110223 (2022)). Therefore, there is still an urgent need to develop a novel chemical modification method with advantages such as high yield, wide application range, and maintenance of a configuration of a modified amino acid residue.

Based on the status quo of the above-mentioned technologies, the inventors of the present application have developed a labeling-activation-desulfurization-addition (LADA) strategy to enable the construction of C-C bonds on polypeptides and proteins. The present disclosure has developed a novel method for visible light-mediated chemical modification of a protein with a cysteine residue as a reaction site and carbon-carbon bond construction as a key according to a sulfhydryl removal/free radical addition reaction strategy. This method can be used in fields such as the chemical transformation for proteins, the development of ADCs, and the development of degrader-antibody conjugates.

### CONTENT OF THE INVENTION

An objective of the present disclosure is to provide a method for visible light-mediated chemical modification of a polypeptide and protein based on cysteine. This method can mildly achieve the construction of a carbon-carbon bond under physiological conditions, and can be used in fields such as chemical transformation for proteins, development of ADCs, and development of degrader-antibody conjugates.

A principle of the LADA strategy of the present disclosure is based on cysteine residues and a sulfhydryl removal/addition reaction strategy. Specifically, a reaction is allowed with an activating reagent to selectively activate sulfhydryl (selectively labeling and activation of cysteine residues), then sulfhydryl removal (desulfurization) is conducted under photocatalysis, and then a generated free radical is subjected to a double bond addition reaction (radical addition) to achieve chemical modification for a polypeptide and protein. Specifically, a cysteine residue (I) is subjected to a substitution reaction (such as a S_{N}Ar reaction) with an activating reagent (II, RX) to generate a free radical precursor A *in situ,* then aryl thiol removal is conducted under light induction, and a generated free radical intermediate is allowed to react with an olefin or an alkyne (III), such that sulfhydryl removal and carbon-carbon bond construction are achieved to produce a chemical modification product of a polypeptide and protein (IV). The LADA strategy of the present disclosure cleverly avoids the influence on a chiral center, and thus the chirality of an amino acid residue can be retained. The whole design is conducive to well avoiding the occurrence of side reactions, and thus there is a very high efficiency of the entire conversion and a prominent yield. The reactions can be conducted mildly under physiological conditions, and can be suitable for various types of amino acid residues. Therefore, the method has advantages such as high efficiency, wide application range, and maintenance of amino acid residue configurations.

The "alkyl" in the present disclosure refers to a group composed of one or more CH₂ moieties. The alkyl can be linear alkyl or branched alkyl. The alkyl can have heteroatom substitutions in a middle or at an end, such as oxygen, nitrogen, sulfur, fluorine, chlorine, bromine, iodine, boron, silicon, and phosphorus substitutions.

The "alkenyl" in the present disclosure refers to a group with at least one carbon-carbon double bond. The alkenyl can be linear or branched. The alkenyl can have heteroatom substitutions in a middle or at an end, such as oxygen, nitrogen, sulfur, fluorine, chlorine, bromine, iodine, boron, silicon, and phosphorus substitutions.

The "alkynyl" in the present disclosure is a general term for compounds including carbon-carbon triple bonds. The alkyne can have heteroatom substitutions in a middle or at an end, such as oxygen, nitrogen, sulfur, fluorine, chlorine, bromine, iodine, boron, silicon, and phosphorus substitutions.

The "aryl" in the present disclosure refers to a planar ring that has a delocalized π electron system and includes 4n + 2 π electrons, where n is an integer. The aryl can be carbocyclic or heterocyclic, including but not limited to phenyl, naphthyl, pyridyl, thienyl, indolyl, and quinolyl. The aryl can have various types of substituents located at any position of a ring, and can be monosubstituted or polysubstituted.

A total reaction equation of the present disclosure is shown in the following equation: 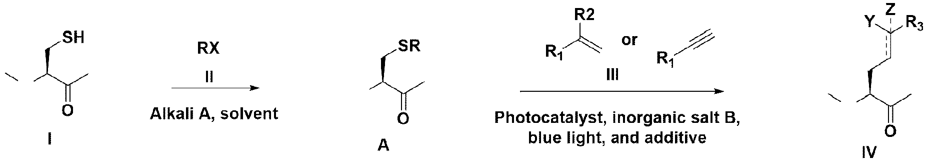 where the compound (I) is selected from any cysteine-containing oligopeptide, polypeptide, or protein; the compound RX (II) is an activating reagent (such as a halide); the compound (III) is a substituted or unsubstituted olefin or alkyne; and the product IV will vary depending on a type of the olefin or alkyne (III) and different additives.

When the halide is a halogenated aromatic ring (ArX), a cysteine residue (I) undergoes a S_{N}Ar reaction with the halogenated aromatic ring (ArX) to generate a free radical precursor A *in situ,* then aryl thiol removal is conducted under light induction, and a generated free radical intermediate undergoes an addition reaction with the olefin (III), such that sulfhydryl removal and carbon-carbon bond construction are achieved to produce a chemical modification product of a polypeptide and protein (IV).

A corresponding reaction equation is shown in the following equation:

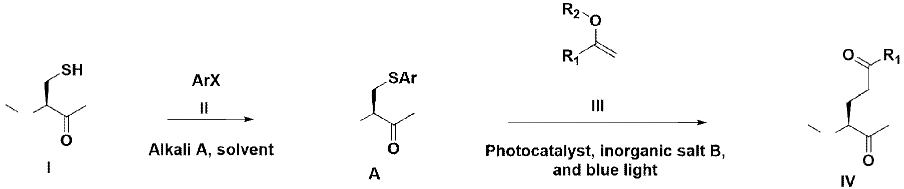

The present disclosure provides a method for visible light-mediated chemical modification of a polypeptide and protein based on cysteine, including the following steps:
S1, allowing a cysteine residue on a cysteine-containing oligopeptide, polypeptide, or protein to undergo a reaction with an activating reagent (such as a halide (RX)) to generate a free radical precursor *in situ*; and
S2', subjecting the free radical precursor to thiol removal under induction of a photocatalyst, and allowing a generated free radical intermediate to undergo an addition reaction with an olefin or an alkyne, such that sulfhydryl removal and carbon-carbon bond construction are achieved to produce a chemical modification product of the polypeptide and protein.

The present disclosure also provides a method for visible light-mediated chemical modification of a polypeptide and protein based on cysteine, including the following steps:
S1', allowing a cysteine residue on a cysteine-containing oligopeptide, polypeptide, or protein to undergo a S_{N}Ar reaction with a halogen or halogenoid-substituted aromatic hydrocarbon to generate a free radical precursor *in situ*; and
S2', subjecting the free radical precursor to aryl thiol removal under light induction, and allowing a generated free radical intermediate to undergo an addition reaction with an olefin, such that sulfhydryl removal and carbon-carbon bond construction are achieved to produce a chemical modification product of the polypeptide and protein.

In the S1, an amount ratio of the cysteine-containing oligopeptide, polypeptide, or protein to the activating reagent (such as a halogen or halogenoid-substituted aromatic hydrocarbon) can be in a very wide range of 1:1 to 1:100,000, such as 1:10, 1:50, 1:100, 1:200, 1:300, 1:500, 1:1,000, 1:2,000, 1:5,000, 1:8,000, 1:10,000, 1:20,000, 1:50,000, 1:70,000, and 1:90,000. In the S2, an amount ratio of the free radical intermediate to the olefin or alkyne can also be in a very wide range of 1:1 to 1:100,000, such as 1:10, 1:50, 1:100, 1:200, 1:300, 1:500, 1:1,000, 1:2,000, 1:5,000, 1:8,000, 1:10,000, 1:20,000, 1:50,000, 1:70,000, and 1:90,000. For the modification of a protein, a very large amount of a small-molecule compound is generally adopted to promote a high reaction yield.

As an embodiment, the cysteine residue is at a C-terminus and/or an N-terminus of the oligopeptide, polypeptide, or protein and/or in a middle of a peptide chain.

In some specific embodiments, the cysteine-containing oligopeptide, polypeptide, or protein is selected from the following compounds: a compound Cys-Boc, a compound Cys-Trp, a compound Cys-Met, a compound Cys-His, a compound Cys-Phe, a compound Cys-Tyr, a compound Cys-Arg, a compound Cys-Ser, a compound Cys-Ala, a compound Cys-Leu, a compound Cys-Boc-Glu, a compound Cys-Lys, a compound Cys-Pro, glutathione, histone H3, and human histone eH3.1.

As an embodiment, the activating reagent is a substituted aromatic or heteroaromatic hydrocarbon including a leaving group; and the leaving group includes a halogen and a halogenoid. In some embodiments, a cysteine residue on a cysteine-containing oligopeptide, polypeptide, or protein is allowed to undergo a S_{N}Ar reaction with a halogen or halogenoid-substituted aromatic hydrocarbon to generate a free radical precursor *in situ,* and the free radical precursor is subjected to aryl thiol removal under light induction.

As an embodiment, the activating reagent is selected from pyrimidine, 1,3,5-triazine, thiazole, imidazole, oxazole, benzoxazole, benzothiazole, and benzimidazole that are substituted with a leaving group such as a halogen and a halogenoid, or is selected from electron-deficient aromatic ring compounds 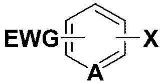 , where X represents a leaving group and the leaving group includes a halogen and a halogenoid; A represents a carbon atom or a nitrogen atom; and EWG represents an electron-withdrawing group or many electron-withdrawing groups. A can be carbon, indicating an electron-deficient benzene ring. A can also be nitrogen, indicating electron-deficient pyridine.

As an embodiment, the halogen includes fluorine, chlorine, bromine, and iodine. The halogenoid includes cyano, thiocyano, selenium cyano, thioether, a sulfone group, and trifluoromethanesulfonic acid (OTf).

As an embodiment, EWG represents an electron-withdrawing group, including but not limited to fluorine, chlorine, bromine, iodine, carbonyl, carboxyl, an ester group, amido, nitro, polyfluoroalkyl, sulfonyl, sulfonamido, a sulfonyl ester group, cyano, a tertiary amine cation, and other substituents with an electron-withdrawing ability. There can be one or more electron-withdrawing groups.

As an embodiment, the activating reagent is selected from one of compounds with the following structural formulas: 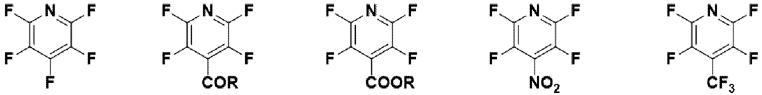 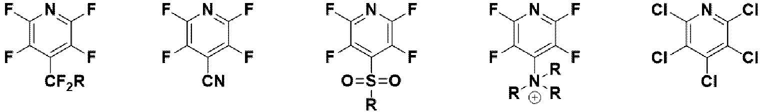 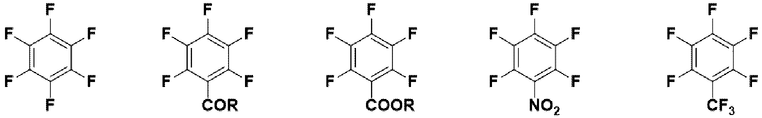 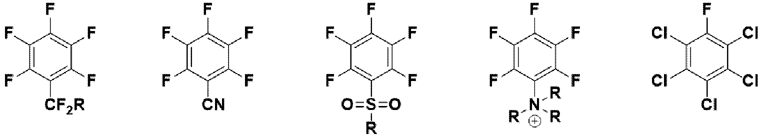 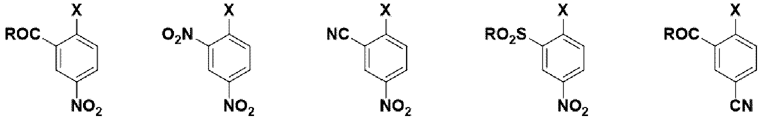 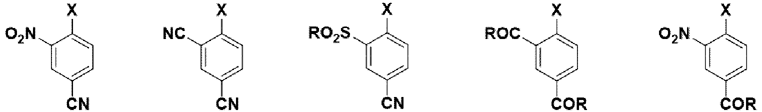 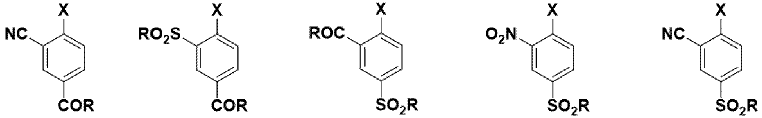 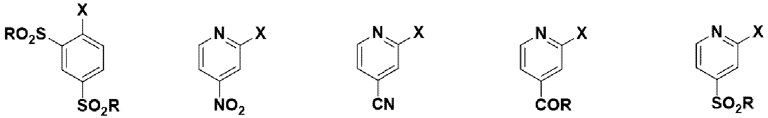 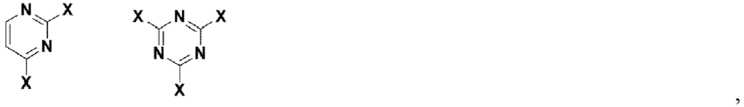 where X represents a halogen or a halogenoid; the halogen includes fluorine, chlorine, bromine, and iodine and the halogenoid includes cyano, thiocyano, selenium cyano, a thioether group, a sulfone group, and a trifluoromethanesulfonly group (OTf); and R is alkyl, aryl, or a heterocycle. In some embodiments, R is C₁-C₁₀ alkyl, 5-10 membered aryl, or 5-10 membered heterocycle.

As an embodiment, the halogen or halogenoid-substituted aromatic hydrocarbon is selected from one of compounds with the following structural formulas: 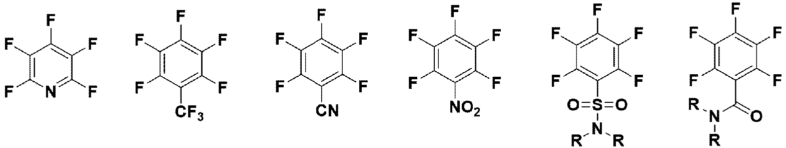 , where R is C₁-C₁₀ alkyl or 5-10 membered aryl.

Compounds with the last two structural formulas can be:

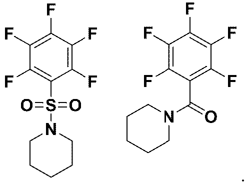

As an embodiment, the activating reagent may also be a salt, such as salts with the following structures: 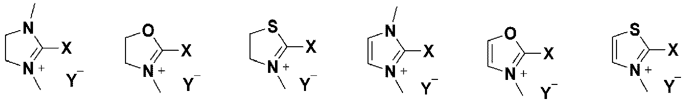 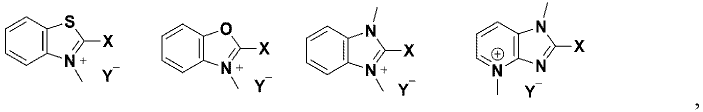 where X is a leaving group such as halogen, thioether, sulfone, and trifluoromethanesulfonly groups (OTf), as shown in the following formulas; and the matching anion Y⁻ includes Cl⁻, Br⁻, I⁻, BF₄⁻, PF₆⁻, OTf⁻, etc.:

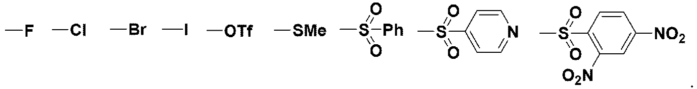

The olefin is a monosubstituted olefin, a disubstituted olefin, or a trisubstituted olefin and is preferably selected from compounds with the following structural formulas: 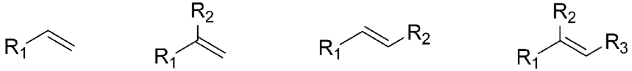 , where R₁, R₂, and R₃ each are aryl, alkyl, alkenyl, or alkynyl.

As an embodiment, the olefin is an silyl enol ether olefin. The olefin is selected from compounds with the following structural formulas: 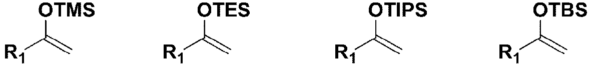 , where R₁ is alkyl and aryl without or with a substituent (referring to a C₁ to C₅₀₀ small-molecule organic compound fragment). During the addition reaction, a functional group or a macromolecular fragment is introduced through the R₁ group; and the functional group includes one or more of hydroxyl, a carboxyl-ether bond, alkenyl, alkynyl, azido, alkynyl, sulfo, amino, halogen, carboxyl, and an ester group, and the macromolecular fragment includes one or more of an unnatural amino acid group, a fluorophore group, a drug molecule group, SiRNA, an antibody, and a ligand binding to an E3 ubiquitin ligase.

As an embodiment, the olefin (III) may also be an enol ester, enamine, or terminal olefin. The alkyne is an aryl alkyne or an aliphatic alkyne, and is preferably a terminal alkyne. The terminal olefin includes a monosubstituted olefin and a bisubstituted terminal olefin.

As an embodiment, the olefin (III) is as follows: 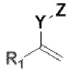 , where R₁ is aryl, alkyl, alkenyl, or alkynyl; Y can be an oxygen atom or NH; Z can be silyl, such as trimethylsilyl (TMS), triethylsilyl, triisopropylsilyl, tert-butyldimethylsilyl, and tert-butyldiphenylsilyl; and Z can also be acyl, including acetyl, trimethylacetyl (Piv), benzoyl, isopropoxycarbonyl, tert-butoxycarbonyl (Boc), benzyloxycarbonyl (Cbz), methanesulfonyl (Ms), p-toluenesulfonyl (Ts), etc. In some embodiments, R₁ is phenyl, naphthyl, thienyl, 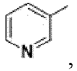 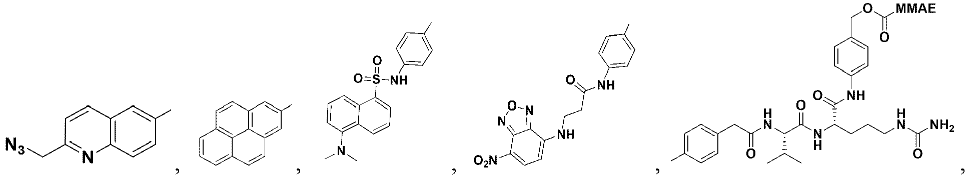 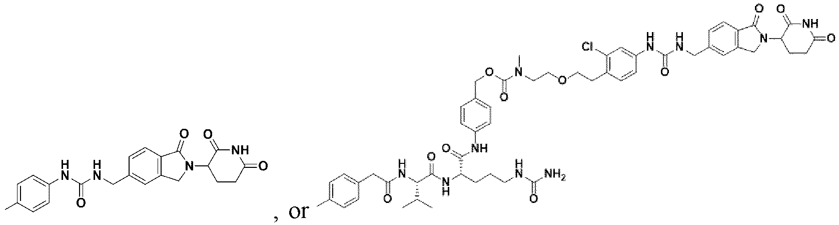 , or is phenyl substituted with halogen, CH₃CH₂NH-, N₃CH₂-, 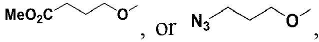 or is naphthyl substituted with CH₃O- or (CH₃)₂N-.

In this case, a general reaction equation is shown in the following equation:

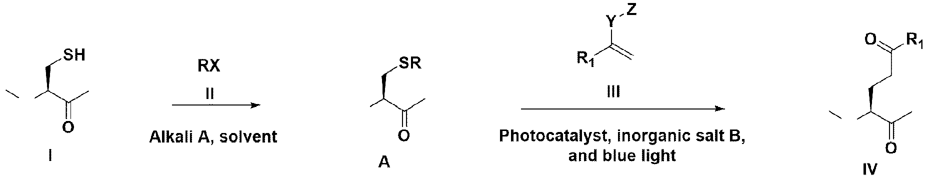

As another embodiment, the olefin (III) is as follows: 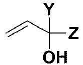 , where Y and Z each are aryl, alkyl, alkenyl, or alkynyl. In this case, a rearrangement reaction takes place, and a general reaction equation is shown in the following equation:

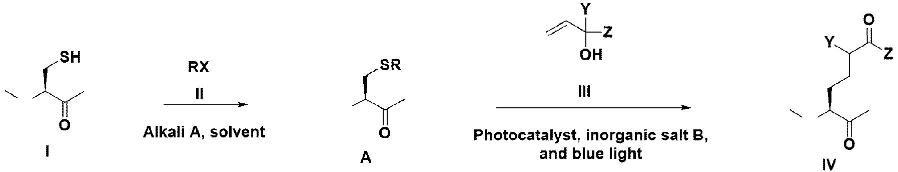

As another embodiment, the olefin (III) is a disubstituted terminal olefin: 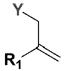 , where R₁ is aryl, alkyl, alkenyl, or alkynyl; and Y can be Cl, Br, I, a sulfone group, polysubstituted silyl, a polysubstituted tin group, etc. The polysubstituted silyl includes TMS, triethylsilyl, triisopropylsilyl, tert-butyldimethylsilyl, and tert-butyldiphenylsilyl. The polysubstituted tin group includes a trimethyltin group, a tributyltin group, a triallyltin group, and a triphenyltin group. In this case, Y is removed to produce an olefin product. A general total reaction equation is shown in the following equation:

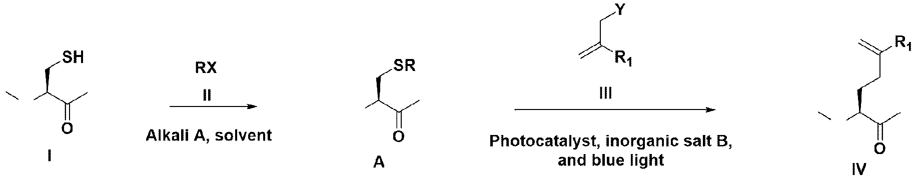

As another embodiment, the olefin (III) is as follows: 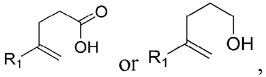 where R₁ is aryl, alkyl, alkenyl, or alkynyl. In this case, cyclization occurs to produce a fivemembered lactone ring or an ether ring. A general reaction equation is shown in the following equation:

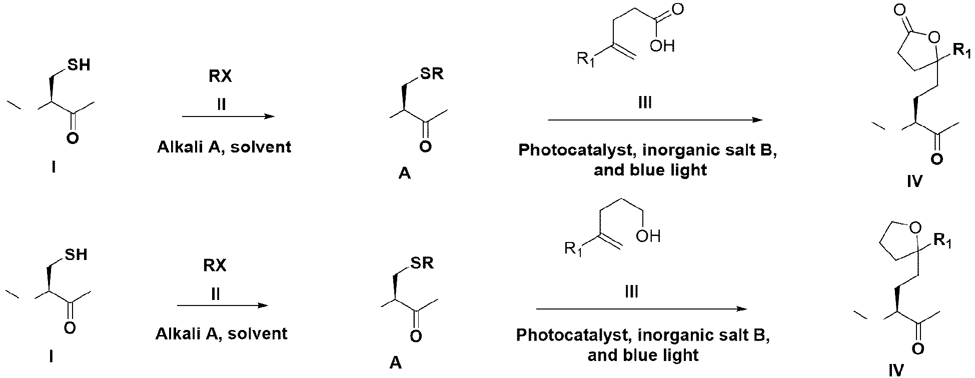

As another embodiment, the olefin (III) is as follows: 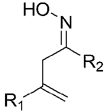 , where R₁ and R₂ each are aryl, alkyl, alkenyl, or alkynyl. In this case, cyclization takes place, and a general reaction equation is shown in the following equation:

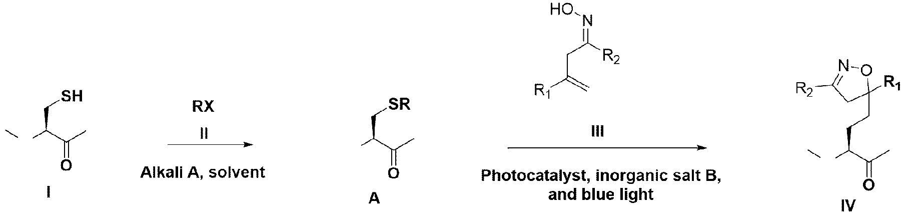

As another embodiment, the olefin (III) is as follows: 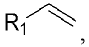 R₁ is aryl and heteroaryl. In this case, the following product is produced.

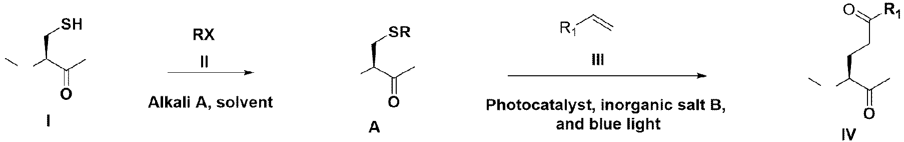

As another embodiment, the olefin (III) is an electron-deficient olefin: 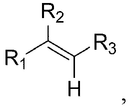 where at least one of R₁ and R₂ includes an electron-withdrawing group such as carbonyl, carboxyl, an ester group, amido, nitro, trifluoromethyl, polyfluoroalkyl, sulfonyl, sulfonamido, a sulfonyl ester group, and cyano; and R₃ is aryl, alkyl, alkenyl, or alkynyl. In this case, a reducing reagent such as thiophenol is added to produce an alkyl product.

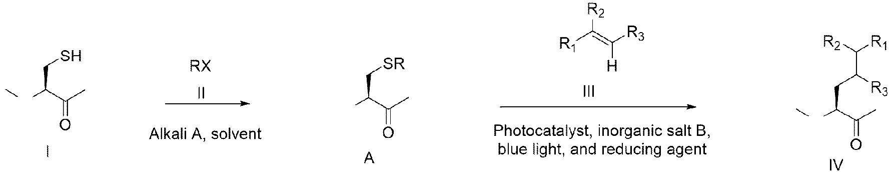

As an embodiment, a functional group or a macromolecular fragment is introduced through at least one of the R₁, R₂, Y, and Z groups; and the functional group includes one or more of hydroxyl, an ether bond, alkenyl, alkynyl, azido, sulfo, amino, halogen, carboxyl, an ester group, and amido, and the macromolecular fragment includes one or more of an unnatural amino acid group, a fluorophore group, a drug molecule group, SiRNA, an antibody, and a ligand binding to an E3 ubiquitin ligase.

As an embodiment, a functional group or a macromolecular fragment is introduced through at least one of the R₁, R₂, Y, and Z groups. The olefin has one of the following structural formulas, where linker represents a (C₁-C₂₀₀) small-molecule organic compound fragment for linking, azide represents azido, alkyne represents alkynyl, flurophore represents a fluorophore group, drug represents a drug molecule group, E3 ligase ligand represents a ligand binding to an E3 ubiquitin ligase, and RNA represents an RNA nucleotide:

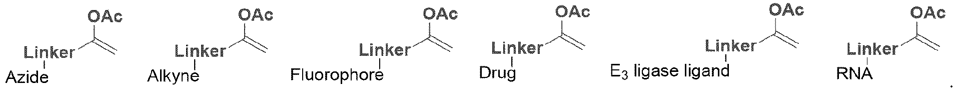

As an embodiment, the olefin is preferably selected from one of compounds with the following structural formulas:

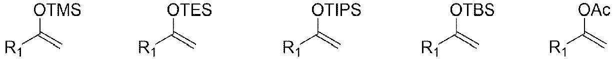

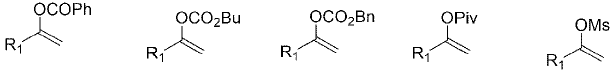

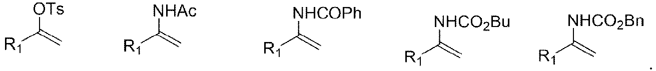

R₁ in the above compound (III) is aryl, alkyl, alkenyl, or alkynyl. In some embodiments, R₁ is alkyl and aryl without or with a substituent. During the addition reaction, a functional group or a macromolecular fragment is introduced through the R₁ group; and the functional group includes one or more of hydroxyl, an ether bond, alkenyl, alkynyl, azido, sulfo, amino, halogen, carboxyl, an ester group, and amido, and the macromolecular fragment includes one or more of an unnatural amino acid group, a fluorophore group, a drug molecule group, SiRNA, an antibody, and a ligand binding to an E3 ubiquitin ligase.

In the above structural formulas of olefins, alkyl as a substituent is (C₁-C₂₀₀) linear or branched alkyl, or is (C₁-C₂₀₀) linear or branched alkyl with a heteroatom substitution in a middle or at an end, and the heteroatom substitution includes a substitution with one or more of oxygen, nitrogen, sulfur, fluorine, chlorine, bromine, iodine, boron, silicon, and phosphorus.

In the above structural formulas of olefins, aryl as a substituent is (C₁-C₂₀₀) carbocyclic or heterocyclic aryl, or is carbocyclic or heterocyclic aryl with a substituent, where the substituent is located at any position of a ring and there is monosubstituted or polysubstituted; the aryl includes, but is not limited to, phenyl, naphthyl, pyridyl, thienyl, indolyl, and quinolyl; and the substituent includes, but is not limited to, (C₁-C₂₀₀) alkyl, (C₁-C₂₀₀) alkenyl, (C₁-C₂₀₀) alkynyl, halogen, nitro, an ester group, amido, amino, hydroxyl, sulfonamido, an ether group, or a thioether group.

In the above structural formulas of olefins, alkenyl as a substituent is (C₁-C₂₀₀) linear or branched alkenyl or is (C₁-C₂₀₀) linear or branched alkenyl with a heteroatom substitution in a middle or at an end, and the heteroatom substitution includes oxygen, nitrogen, sulfur, fluorine, chlorine, bromine, iodine, boron, silicon, and phosphorus substitutions.

In the above structural formulas of olefins, alkynyl as a substituent is an (C₁-C₂₀₀) alkyne or is an (C₁-C₂₀₀) alkyne with a heteroatom substitution in a middle or at an end, and the heteroatom substitution includes a substitution with one or more of oxygen, nitrogen, sulfur, fluorine, chlorine, bromine, iodine, boron, silicon, and phosphorus.

The C₁-C₂₀₀ includes C₁-C₁₀, C₁₀-C₂₀, C₂₀-C₃₀, C₃₀-C₄₀, C₄₀-C₅₀, C₅₀-C₆₀, C₆₀-C₇₀, C₇₀-C₈₀, C₈₀-C₉₀, C₉₀-C₁₀₀, C₁₁₀-C₁₂₀, C₁₂₀-C₁₃₀, C₁₃₀-C₁₄₀, C₁₄₀-C₁₅₀, C₁₅₀-C₁₆₀, C₁₆₀-C₁₇₀, C₁₇₀-C₁₈₀, C₁₈₀-C₁₉₀, and C₁₉₀-C₂₀₀, for example.

As an embodiment of the present disclosure, the olefin is an enol ester, enamine, or terminal olefin. The alkyne is an aryl alkyne or an aliphatic alkyne, and is preferably a terminal alkyne. The aryl alkyne is C₁-C₂₀₀, and the aliphatic alkyne is C₁-C₂₀₀.

As another embodiment, during the addition reaction with the olefin or the alkyne, some additives (nucleophiles, NuH) can also be added, such that different products can be produced. For example, the additives include Hantzsch ester, water, TMS cyanide (TMSCN), an azide, hexamethyldisilane, dihydropyridine, triethylsilane, thiophenol, etc., which correspond to different products. A general reaction equation is shown in the following equation, where R₁ is alkyl and aryl:

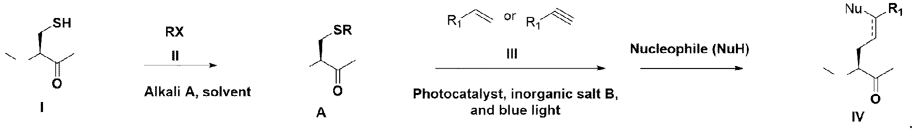

It should be noted that there are abundant types of olefins or alkynes III and additives (nucleophiles, NuH) that can participate in the reaction, which cannot be described in detail, but all fall within the protection scope of this patent.

For example, when the olefin III involved in the reaction is an electron-deficient olefin, dichlorothiophenol can be added as a hydrogen source, and a product is an alkane, as shown in the following equation:

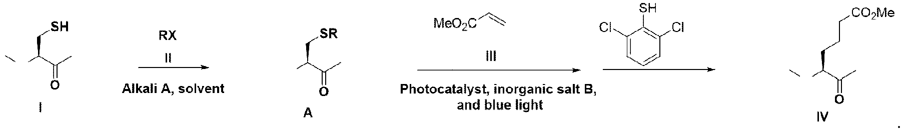

For example, when the alkyne III involved in the reaction is phenylacetylene and the nucleophile is a Hantzsch ester, a product is an olefin (mainly a trans-olefin), as shown in the following equation:

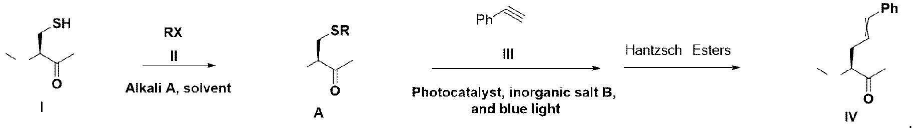

For another example, when the olefin III involved in the reaction is phenylacetylene and the nucleophile is water, a product is a ketone, as shown in the following equation:

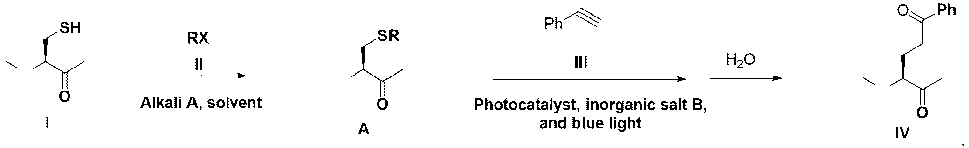

More importantly, in the above addition reaction, various functional groups such as hydroxyl, an ether bond, an olefin, an alkyne, azido, sulfonyl, amino, halogen, carboxyl, and an ester group can be introduced through the olefin or the alkyne. A macromolecular fragment can also be introduced, such as one or more of an unnatural amino acid group, a fluorophore group, a drug molecule group, SiRNA, an antibody, and a ligand binding to an E3 ubiquitin ligase. It should be noted that a desired function can be introduced through aryl, for example, a group such as an alkyne and an azide can be introduced for click chemistry. An unnatural amino acid, a fluorophore, a drug molecule, SiRNA, an antibody, a ligand binding to an E3 ubiquitin ligase, etc. can also be introduced to allow structural modification for a protein, labeling and tracing for a protein, development of ADC, development of a degrader-antibody conjugate, development of an antibody-oligonucleotide conjugate (AOC), development of an antibody-cell conjugate (ACC), development of an antibody-photosensitizer conjugate, etc. Those with a basic chemistry education background can easily understand that desired functional groups and molecular fragments can be introduced through enols, which falls within the protection scope of this patent.

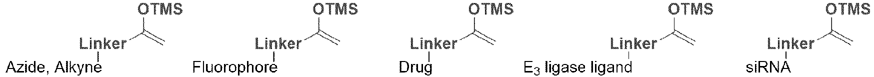

For well understanding, for example, an aromatic ring, a heteroaromatic ring, and an olefin can be introduced through the following olefins:

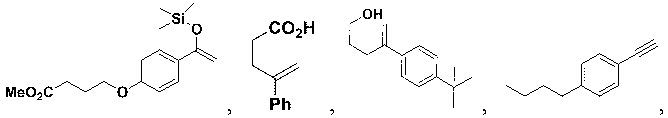

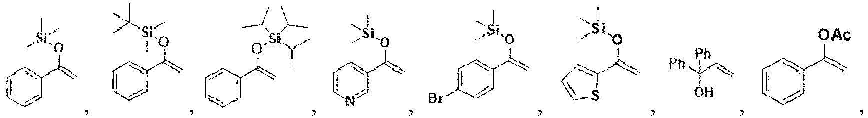

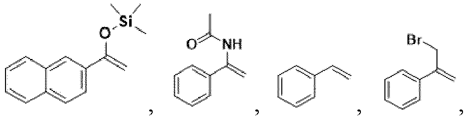

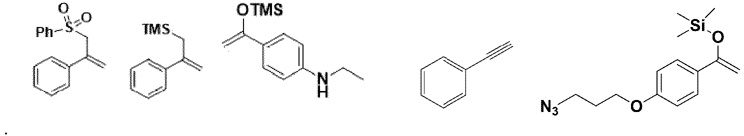

For example, an silyl enol ether can introduce alkynyl and an azide:

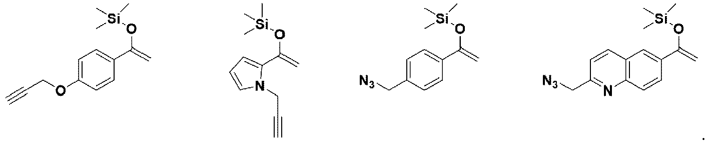

A fluorophore can also be introduced, such as:

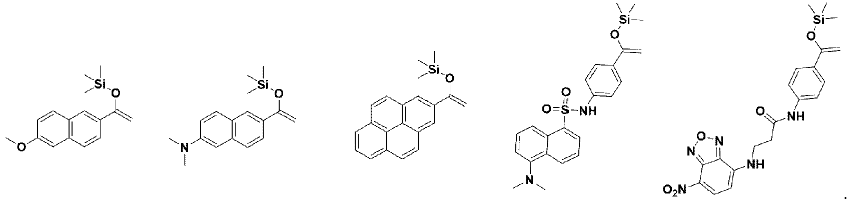

More importantly, a drug molecule can also be introduced to develop antibody-drug conjugate (ADC), as shown in the following formulas, where drug represents monomethyl auristatin E (MMAE), monomethyl auristatin F (MMAF), calicheamicin, duocarmycin, a mertansine derivative DM1, a topoisomerase I inhibitor SN-38 (NK012), a camptothecin (CPT) analogue (deruxtecan (DXd)), or pyrrolobenzodiazepine (PBD):

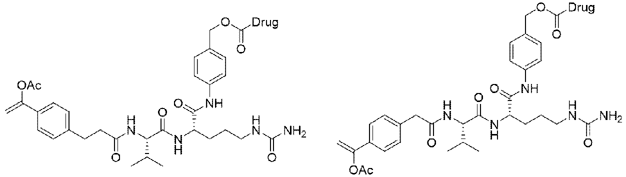

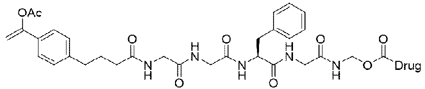

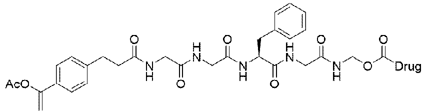

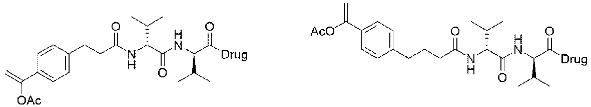

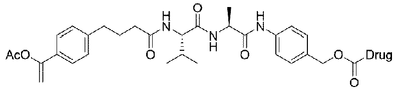

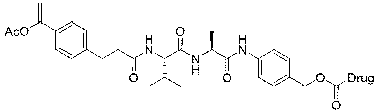

In some embodiments, the microtubule disruptor MMAE is introduced:

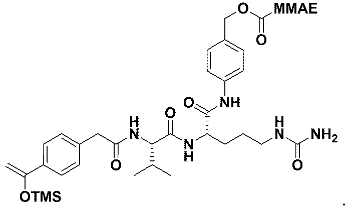

In addition, a ligand for an E3 ubiquitin ligase can also be introduced to develop a degrader-antibody conjugate, such as the following examples:

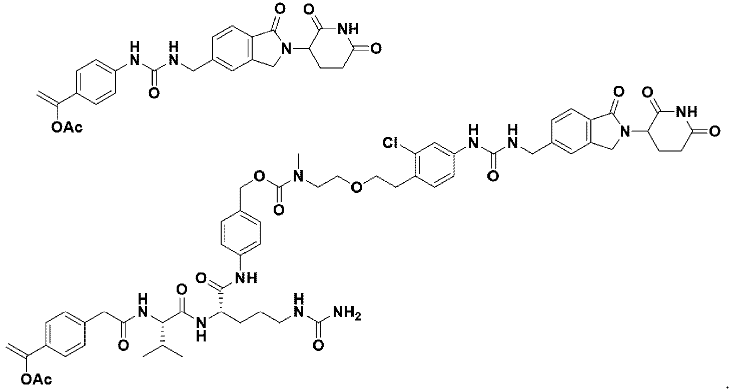

In some embodiments, the following are introduced:

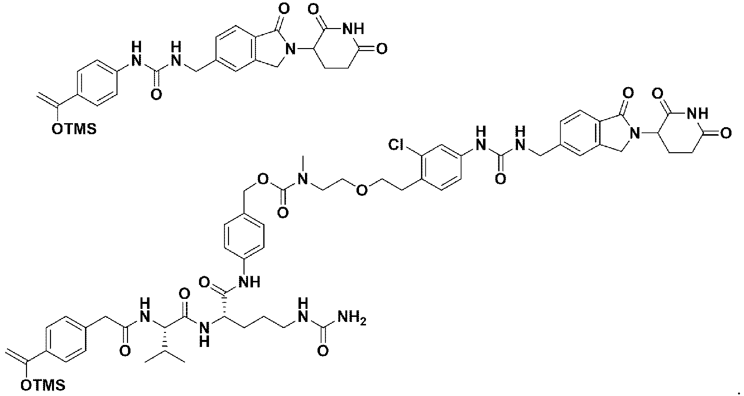

Similarly, antibody-oligonucleotide conjugate (AOC) and antibody-cell conjugate (ACC) can also be developed, which cannot be described in detail, but all fall within the protection scope of this patent.

As an embodiment, in the S1, the substitution reaction (such as a S_{N}Ar reaction) with the activating reagent (RX) is conducted in the presence of an alkali and a solvent.

As an embodiment, the alkali is selected from N,N-diisopropylethylamine (DIPEA), sodium bicarbonate, sodium carbonate, zinc acetate, potassium acetate, or a weakly-alkaline buffer solution.

As an embodiment, the solvent is selected from one of an organic solvent, water, a buffer solution, a mixed solvent of an organic solvent and water, and a mixed solvent of an organic solvent and a buffer solution.

As an embodiment, the organic solvent is selected from dimethyl sulfoxide (DMSO), N,N-dimethylformamide (DMF), tetrahydrofuran (THF), acetonitrile, acetone, and ethyl acetate. The buffer solution is selected from a phosphoric acid, citric acid, carbonic acid, or acetic acid buffer solution.

As an embodiment, in the S2, the reaction is conducted in the presence of the photocatalyst and blue light. During the reaction, an inorganic salt (including an inorganic acid and an inorganic alkali) can be added to further improve a reaction yield. In a special case, an electron donor-acceptor (EDA) complex can also be generated to stimulate generation of a free radical.

As an embodiment, the photocatalyst is selected from a water-soluble Ir or Ru photocatalyst. In a special case, an EDA complex can also be generated to stimulate generation of a free radical under light.

As an embodiment, the photocatalyst is selected from Ir(ppy)₃, Ru(bpm)₃Cl₂, and Ru(bpy)₃Cl₂. The photocatalyst can also be a water-soluble photocatalyst, such as [Ir(sppy)₃]³⁻, [Ir(s^{F}ppy)₃]³⁻, [Ir(s^{dF}ppy)₃]³⁻, and [Ir(s^{CH2}ppy)₃]⁻, with structures shown in the following formulas:

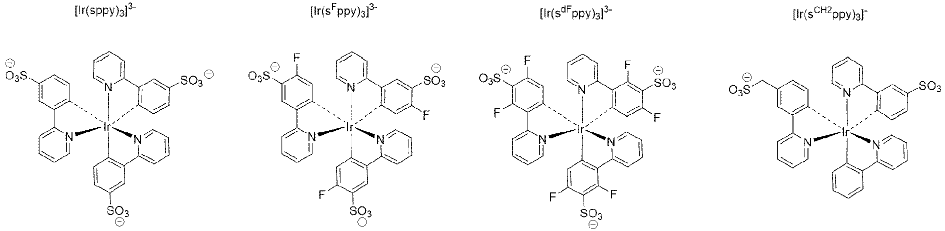

A light source adopted is visible light, preferably blue light, and more preferably Kessil lamp, such as 40 W 440 nm Kessil lamp.

As an embodiment, the inorganic salt is an inorganic alkali or an inorganic acid. The inorganic salt is preferably selected from zinc acetate, zinc trifluoroacetate, sodium carbonate, potassium carbonate, sodium bicarbonate, copper acetate, sodium acetate, potassium acetate, cesium carbonate, zinc chloride, zinc fluoride, etc. In addition, triphenylphosphine can be added, which sometimes will further increase the yield.

Further, a reaction principle of the LADA strategy developed by the present disclosure is explained with a fluoroaromatic hydrocarbon as an electron-deficient halogenated aromatic hydrocarbon/an enol trimethylsilyl ether as an olefin for example, and a route for the principle is as follows:

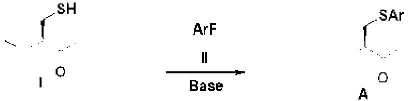

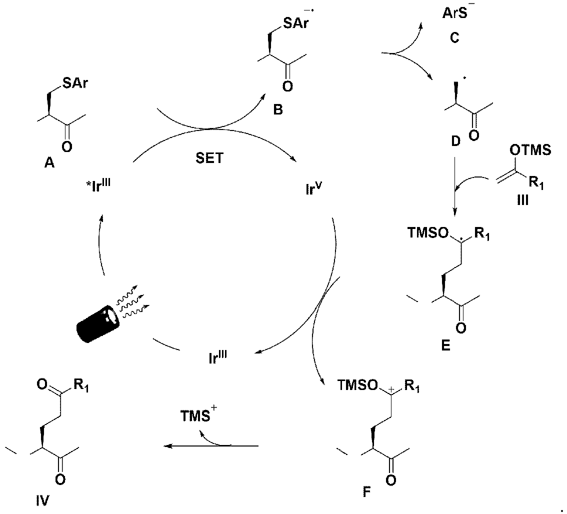

The cysteine residue **I** selectively undergoes a S_{N}Ar reaction with the electron-deficient fluoroaromatic hydrocarbon **II** to generate an intermediate **A** *in situ,* where other functional groups on a polypeptide or protein are not affected. The intermediate **A** receives an electron from an excited photocatalyst to produce a free radical anion **B,** and then an aryl thiophenol anion **C** is removed to produce a free radical intermediate **D.** The free radical intermediate **D** undergoes an addition reaction with the raw material silyl enol ether **III** to produce a free radical intermediate **E.** The free radical intermediate **E** is then oxidized by a photocatalyst to produce an intermediate **F,** and the photocatalyst is reduced to complete a catalytic cycle. The intermediate **F** loses a TMS cation to finally produce a product **IV.** This design strategy cleverly avoids the influence on a chiral center, and thus the chirality of an amino acid residue can be retained. The whole design is conducive to well avoiding the occurrence of side reactions, and thus there is a very high efficiency of the entire conversion and a prominent yield.

The LADA strategy developed by the present disclosure provides a use of the method for chemical modification of a polypeptide and protein based on cysteine described above in structural modification for a polypeptide and protein, labeling and tracing for a protein, development of ADC, development of a degrader-antibody conjugate, development of AOC, development of ACC, or development of an antibody-photosensitizer conjugate.

Compared with the prior art, the LADA strategy developed by the inventors has the following effects:
1) A polypeptide or protein modified by the present disclosure has diversity, and can be expanded to an oligopeptide, polypeptide, and protein. A position of a cysteine residue in a polypeptide or protein is not affected, and can be expanded to a C-terminus, an N-terminus, and a middle of a peptide chain. The present disclosure is highly selective for a sulfhydryl modification of a cysteine residue, and does not affect other natural amino acid residues.
2) The method has prominent functional group compatibility for the compound (III), including compatibility for hydroxyl, an ether bond, an olefin, an alkyne, azido, sulfo, amino, halogen, an ester group, carboxyl, etc. The method achieves the highly-selective sulfhydryl removal-carbon-carbon bond modification for cysteine residues for the first time. The method overcomes the limitation in the traditional polypeptide and protein modification that an amino acid is incompatible with a functional group, and has a promising application prospect.
3) The method of the present disclosure involves mild conditions and simple and convenient operations, and can allow the selective modification for complex polypeptides and proteins through one-pot synthesis.
4) A modification product produced by the present disclosure retains the original configuration, which avoids the defects of the methods of B. G. Davis and Hee-Sung Park, and has great advantages.
5) The method of the present disclosure can construct a carbon-carbon bond on a polypeptide and protein. The carbon-carbon bond has much better stability than a carbon-sulfur bond commonly used in ADCs, and can avoid the toxicity caused by carbon-sulfur bond breaking. Therefore, the method of the present disclosure has a wide application prospect in the development of ADCs, the development of degrader-antibody conjugates, the development of AOCs, the development of ACCs, or the development of antibody-photosensitizer conjugates.

### DESCRIPTION OF THE DRAWINGS

Other features, objectives, and advantages of the present disclosure will become more apparent by reading the detailed description of non-limiting embodiments with reference to the following accompanying drawings.
FIG. 1 is a hydrogen nuclear magnetic resonance spectrum of a compound DP-02;
FIG. 2 is a carbon nuclear magnetic resonance spectrum of a compound DP-02;
FIG. 3 is a hydrogen nuclear magnetic resonance spectrum of a compound DP-05;
FIG. 4 is a carbon nuclear magnetic resonance spectrum of a compound DP-05;
FIG. 5 is a high-performance liquid chromatography (HPLC) spectrum of a product from a chemical modification of histone H3;
FIG. 6 is a mass spectrometry spectrum of the product from the chemical modification of histone H3;
FIG. 7 is an HPLC spectrum of a product from a chemical modification of human histone eH3.1;
FIG. 8 is a mass spectrometry spectrum of the product from the chemical modification of human histone eH3.1;
FIG. 9 is an HPLC spectrum of a polypeptide compound DP-15;
FIG. 10 is a mass spectrometry spectrum of the polypeptide compound DP-15;
FIG. 11 is an HPLC spectrum of a polypeptide compound DP-16;
FIG. 12 is a mass spectrometry spectrum of the polypeptide compound DP-16;
FIG. 13 is an HPLC spectrum of a polypeptide compound DP-17;
FIG. 14 is a mass spectrometry spectrum of the polypeptide compound DP-17;
FIG. 15 is an HPLC spectrum of a polypeptide compound DP-18;
FIG. 16 is a mass spectrometry spectrum of the polypeptide compound DP-18;
FIG. 17 is an HPLC spectrum of a polypeptide compound DP-19;
FIG. 18 is a mass spectrometry spectrum of the polypeptide compound DP-19;
FIG. 19 is an HPLC spectrum of a polypeptide compound DP-20;
FIG. 20 is a mass spectrometry spectrum of the polypeptide compound DP-20;
FIG. 21 shows the instability of C-S bond-containing ADC in plasma;
FIG. 22 shows the stability of a C-S bond-containing compound 1 in plasma;
FIG. 23 shows the stability of a compound DP-04 in plasma;
FIG. 24 shows the stability of a C-S bond-containing compound 2 in plasma; and
FIG. 25 shows the stability of a compound DP-11 in plasma.

### SPECIFIC IMPLEMENTATIONS

The present disclosure is described in detail below with reference to examples. The following examples will help those skilled in the art further understand the present disclosure, but will not limit the present disclosure in any way. It should be noted that those with ordinary skill in the field can further make several modifications and improvements without departing from the idea of the present disclosure. These all fall within the protection scope of the present disclosure.

### Example 1 Preparation of a compound AA-01 with pentafluoropyridine

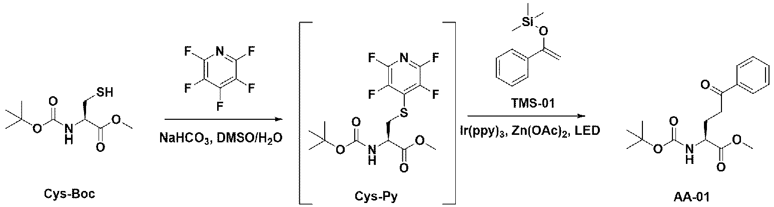

A compound Cys-Boc (42.0 mg, 0.18 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. 6 mL of DMSO was added, and anhydrous Zn(OAc)₂ (22.0 mg, 0.12 mmol), Ir(ppy)₃ (2.0 mg, 0.003 mmol), and an silyl enol ether TMS-01 (0.16 mL, 0.8 mmol) were added. Nitrogen replacement was conducted 3 times. Stirring was conducted for 14 h under the irradiation of a blue light-emitting diode (LED). At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (53 mg, yield: 91%). ¹H NMR (400 MHz, Chloroform-d) δ 7.93 (d, *J* = 7.7 Hz, 2H), 7.55 (t, *J* = 7.4 Hz, 1H), 7.44 (t, *J* = 7.6 Hz, 2H), 5.15 (d, *J* = 8.6 Hz, 1H), 4.38 (d, *J* = 7.4 Hz, 1H), 3.73 (s, 3H), 3.21-2.95 (m, 2H), 2.39-2.22 (m, 1H), 2.15-1.93 (m, 1H), 1.40 (s, 9H). An optical rotation was [α]_{D}²⁰ = 14.7 (C = 1.0, CHCl₃), which was consistent with the report in the literature, indicating that a configuration of a chiral center of an amino acid was retained.

An intermediate Cys-Py could be separated and purified, and nuclear magnetic resonance data of the intermediate was as follows:

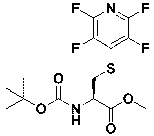

¹H NMR (400 MHz, Chloroform-*d*) δ 5.29 (d, J = 7.9 Hz, 1H), 4.63 (q, J = 5.5 Hz, 1H), 3.81 - 3.76 (m, 1H), 3.75 (s, 3H), 3.45 (dd, *J =* 14.3, 5.2 Hz, 1H), 1.40 (s, 9H).

### Example 2 Preparation of a compound AA-01 with octafluorotoluene

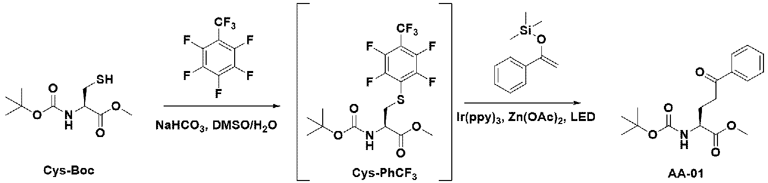

A compound Cys-Boc (42.0 mg, 0.18 mmol) was dissolved in 4 mL of DMSO, 0.1 mL of water was added, then NaHCO₃ (18.0 mg, 0.21 mmol) was added, and then 50 mg of octafluorotoluene was added. Stirring was conducted at room temperature to allow a reaction for 20 min. 6 mL of DMSO was added, and anhydrous Zn(OAc)₂ (22.0 mg, 0.12 mmol), Ir(ppy)₃ (2.0 mg, 0.003 mmol), and an silyl enol ether TMS-01 (0.09 mL, 0.45 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 75%).

### Example 3 Preparation of a compound AA-01 with pentafluorobenzonitrile

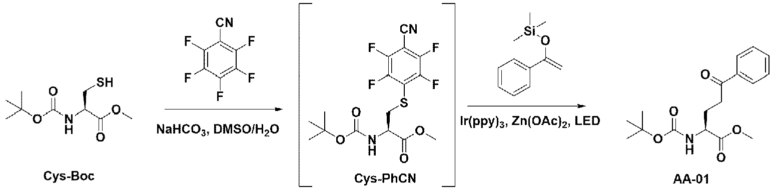

A compound Cys-Boc (42.0 mg, 0.20 mmol) was dissolved in 4 mL of DMSO, 0.1 mL of water was added, then NaHCO₃ (18.0 mg, 0.21 mmol) was added, and then 41 mg of pentafluorobenzonitrile was added. Stirring was conducted at room temperature to allow a reaction for 20 min. 6 mL of DMSO was added, and anhydrous Zn(OAc)₂ (22.0 mg, 0.12 mmol), Ir(ppy)₃ (2.0 mg, 0.003 mmol), and an silyl enol ether TMS-01 (0.09 mL, 0.45 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 55%).

### Example 4 Preparation of a compound AA-01 with a benzimidazolium salt

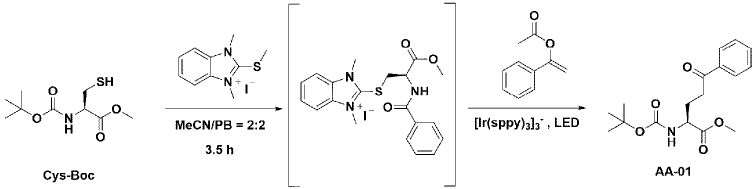

A compound Cys-Boc (42.0 mg, 0.20 mmol) was dissolved in 2 mL of acetonitrile and 2 mL of phosphate-buffered saline (pH: 7.5), and then an activating reagent of 1,3-dimethyl-2-(methylthio)-1H-benzo[d]imidazol-3-iumiodide (127.6 mg, 0.40 mmol) was added. Stirring was conducted at room temperature to allow a reaction for 3.5 h. Then, a water-soluble photocatalyst [Ir(sppy)₃]³⁻ (3.0 mg) and an enol ester **OAc** (192.0 mg, 1.2 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 85%).

### Example 5 Preparation of a compound AA-01 with OTBS

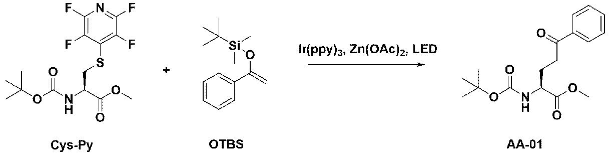

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether OTBS (47.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 80%).

### Example 6 Preparation of a compound AA-01 with OTIPS

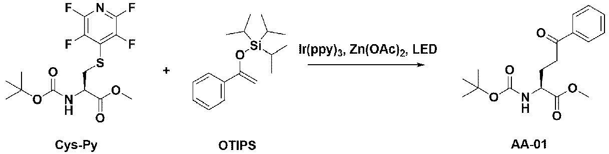

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether OTIPS (55.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 64%).

### Example 7 Preparation of a compound AA-02

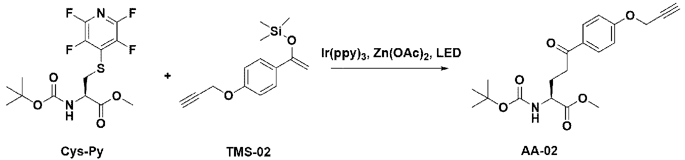

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-02 (123.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-02 (yield: 85%). ¹H NMR (400 MHz, Chloroform-*d*) δ 7.97-7.91 (m, 2H), 7.01 (d, *J=* 8.7 Hz, 2H), 5.24 (d, *J* = 8.3 Hz, 1H), 4.76 (d, *J* = 2.4 Hz, 2H), 4.38 (q, *J =* 7.6 Hz, 1H), 3.74 (s, 3H), 3.12-2.96 (m, 2H), 2.57 (t, *J =* 2.3 Hz, 1H), 2.33-2.24 (m, 1H), 2.18-2.03 (m, 1H), 1.42 (s, 9H). ¹³C NMR (101 MHz, Chloroform-*d*) δ 197.32, 172.85, 161.29, 155.40, 130.41, 130.16, 114.57, 79.89, 77.68, 76.14, 55.79, 53.10, 52.30, 34.15, 28.21, 26.95.

### Example 8 Preparation of a compound AA-03

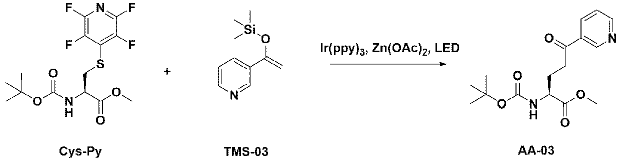

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-03 (97.0 mg, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-03 (yield: 81%). ¹H NMR (400 MHz, Chloroform-d) δ 9.16 (d, *J =* 2.2 Hz, 1H), 8.79 (dd, *J* = 5.0, 1.7 Hz, 1H), 8.23 (dt, *J =* 8.0, 2.0 Hz, 1H), 7.43 (dd, *J =* 8.0, 4.9 Hz, 1H), 5.29 - 5.22 (m, 1H), 4.46-4.34 (m, 1H), 3.76 (s, 3H), 3.22-3.03 (m, 2H), 2.39-2.32 (m, 1H), 2.14-2.03 (m, 1H), 1.41 (s, 9H). ¹³C NMR (101 MHz, Chloroform-d) δ 197.60, 172.67, 155.40, 153.49, 149.46, 135.26, 131.90, 123.55, 80.05, 52.81, 52.40, 34.70, 28.19, 26.75.

### Example 9 Preparation of a compound AA-04

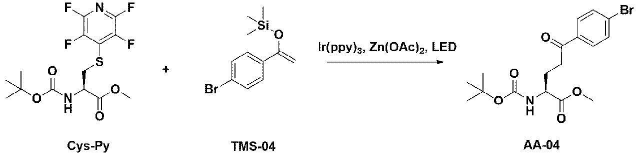

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-04 (136.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-04 (yield: 93%). ¹H NMR (400 MHz, Chloroform-d) δ 8.04 (t, *J* = 1.8 Hz, 1H), 7.84 (dt, *J* = 7.8, 1.4 Hz, 1H), 7.66 (dt, *J* = 7.9, 1.4 Hz, 1H), 7.32 (t, *J =* 7.9 Hz, 1H), 5.18 (d, *J =* 8.3 Hz, 1H), 4.36 (d, *J =* 7.0 Hz, 1H), 3.73 (s, 3H), 3.12-2.95 (m, 2H), 2.36-2.28 (m, 1H), 2.12-2.02 (m, 1H), 1.39 (s, 9H).¹³C NMR (101 MHz, Chloroform-*d*) δ 197.42, 172.74, 155.41, 138.38, 135.97, 131.05, 130.17, 126.50, 122.94, 80.05, 52.90, 52.41, 34.53, 28.22, 26.88.

### Example 10 Preparation of a compound AA-05

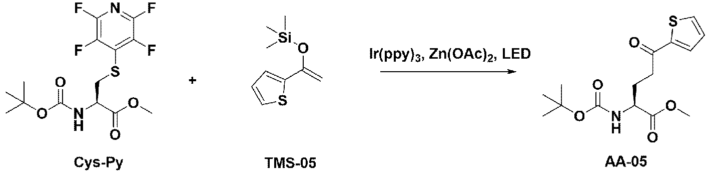

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-05 (139.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-05 (yield: 85%). ¹H NMR (400 MHz, Chloroform-d) δ 7.56 - 7.51 (m, 1H), 7.15 (d, J = 3.5 Hz, 1H), 6.49 (dd, J = 3.6, 1.8 Hz, 1H), 5.15 (d, J = 8.3 Hz, 1H), 4.32 (d, J = 7.6 Hz, 1H), 3.70 (s, 3H), 2.97-2.83 (m, 2H), 2.26- 2.16 (m, 1H), 2.07-1.98 (m, 1H), 1.37 (s, 9H). ¹³C NMR (101 MHz, Chloroform-d) δ 187.94, 172.69, 155.36, 152.42, 146.29, 117.00, 112.18, 79.93, 53.03, 52.31, 34.26, 31.44, 30.08, 28.20, 26.56.

### Example 11 Preparation of a compound AA-6

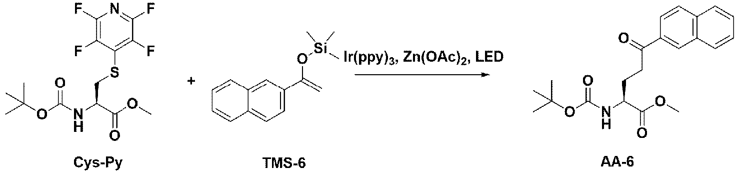

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-6 (121.0 mg, 5.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-6 (yield: 90%). ¹H NMR (400 MHz, Chloroform-d) δ 8.46 (s, 1H), 8.01 (dd, *J =* 8.6, 1.7 Hz, 1H), 7.95 (d, *J =* 8.0 Hz, 1H), 7.90 - 7.84 (m, 2H), 7.57 (dt, *J =* 19.8, 7.2 Hz, 2H), 5.26 (d, *J =* 8.5 Hz, 1H), 4.44 (q, *J =* 7.6 Hz, 1H), 3.76 (s, 3H), 3.31-3.14 (m, 2H), 2.42-2.32 (m, 1H), 2.20-2.09 (m, 1H), 1.42 (s, 9H). ¹³C NMR (101 MHz, Chloroform-d) δ 198.72, 172.87, 155.44, 135.58, 133.96, 132.45, 129.65, 129.51, 128.44, 128.40, 127.71, 126.75, 123.70, 79.97, 53.11, 52.35, 34.54, 28.22, 27.07.

### Example 12 Preparation of a compound AA-7

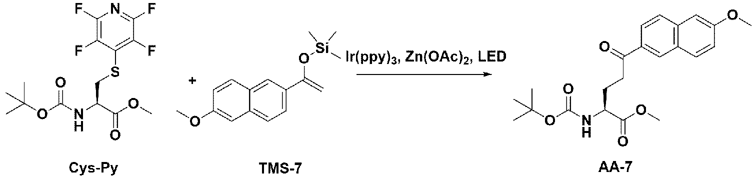

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether TMS-7 (136.0 mg, 2.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-7 (yield: 88%). ¹H NMR (400 MHz, Chloroform-d) δ 8.39-8.35 (m, 1H), 7.97 (dd, *J =* 8.6, 1.7 Hz, 1H), 7.82 (d, *J =* 9.0 Hz, 1H), 7.74 (d, *J* = 8.6 Hz, 1H), 7.18 (dd, *J =* 9.0, 2.5 Hz, 1H), 7.13 (d, *J =* 2.5 Hz, 1H), 5.19 (d, *J =* 8.3 Hz, 1H), 4.40 (d, *J =* 7.3 Hz, 1H), 3.93 (s, 3H), 3.74 (s, 3H), 3.28-3.06 (m, 2H), 2.40-2.26 (m, 1H), 2.12 (dt, *J* = 14.9, 7.5 Hz, 1H), 1.40 (s, 9H).

### Example 13 Preparation of a compound AA-8

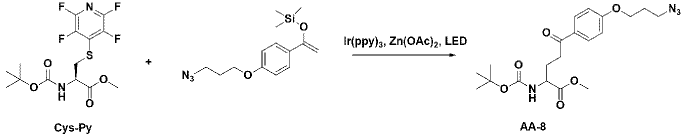

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether (146 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-8 (yield: 95%).

¹H NMR (400 MHz, Chloroform-*d*) δ 7.90 (d, *J =* 8.9 Hz, 2H), 6.90 (d, *J =* 8.9 Hz, 2H), 5.20 (d, *J =* 8.3 Hz, 1H), 4.34 (d, *J =* 6.9 Hz, 1H), 4.09 (t, *J =* 6.0 Hz, 2H), 3.71 (s, 3H), 3.50 (t, *J =* 6.6 Hz, 2H), 3.08 - 2.93 (m, 2H), 2.31 - 2.22 (m, 1H), 2.10 - 2.00 (m, 3H), 1.39 (s, 9H). ¹³C NMR (101 MHz, Chloroform-d) δ 197.31, 172.86, 162.58, 155.41, 130.27, 129.95, 114.16, 79.90, 64.74, 53.16, 52.29, 48.05, 34.13, 28.61, 28.23, 27.03.

### Example 14 Preparation of a compound AA-9

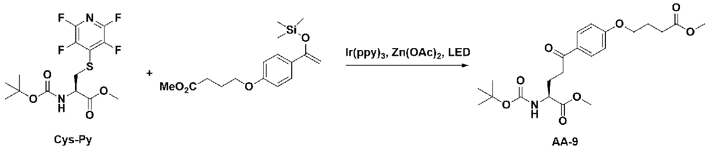

A compound **Cys-Py** (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and a silyl enol ether (146 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-9 (yield: 88%). ¹H NMR (400 MHz, Chloroform-*d*) δ 7.92 (d, *J =* 8.8 Hz, 2H), 6.91 (d, *J =* 8.6 Hz, 2H), 5.27 (d, *J =* 8.3 Hz, 1H), 4.37 (q, *J =* 7.4 Hz, 1H), 4.08 (t, *J =* 6.1 Hz, 2H), 3.74 (s, 3H), 3.70 (s, 3H), 3.04 (qdd, *J =* 17.5, 8.2, 6.3 Hz, 2H), 2.54 (t, *J =* 7.2 Hz, 2H), 2.35 - 2.21 (m, 1H), 2.12 (dp, *J =* 16.2, 7.7, 7.2 Hz, 2H), 1.42 (s, 9H). ¹³C NMR (101 MHz, Chloroform-*d*) δ 197.33, 173.39, 172.88, 162.74, 155.42, 130.23, 129.75, 114.13, 79.86, 66.89, 53.14, 52.29, 51.61, 34.10, 30.31, 28.21, 26.98, 24.39. ESI-MS calcd for C₂₂H₃₁NO₈Na [M+Na]⁺m/z = 460.1942, found:460.1949.

### Example 145 Preparation of a compound AA-01 with an enol ester

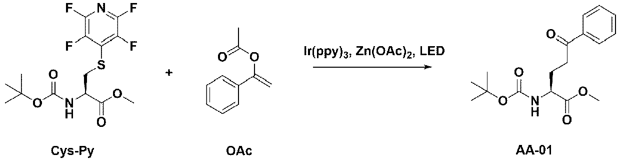

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and an enol ester OAc (81.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 90%).

### Example 16 Preparation of a compound AA-01 with an enamine

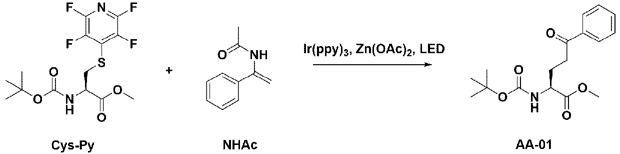

A compound Cys-Py (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), and an enamine NHAc (81.0 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product AA-01 (yield: 80%).

### Example 17 Preparation of a compound AA-01 with styrene

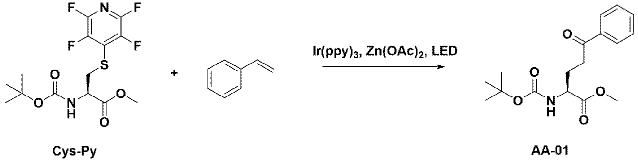

A compound **Cys-Py** (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), styrene (58 µL, 0.5 mmol), and water (0.1 mL) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-01** (yield: 65%).

### Example 18 Preparation of a compound AA-01 with imidazolium tetrafluoroborate for activation

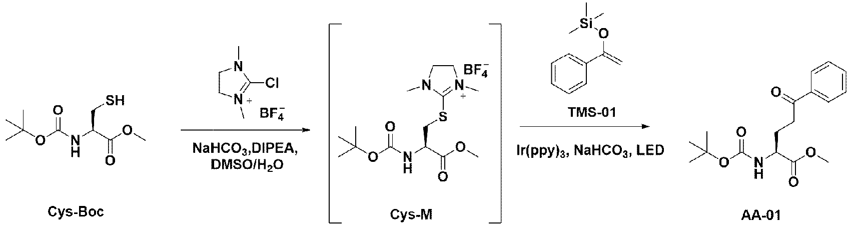

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-01** (yield: 82%).

### Example 19 Preparation of a compound AA-01 with imidazolium chloride for activation

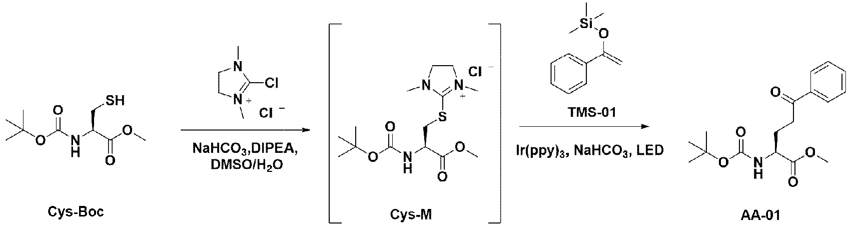

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolinium chloride (37 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-01** (yield: 70%).

### Example 20 Preparation of a compound AA-10 with a salt for activation

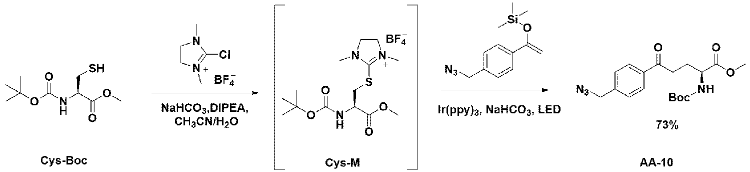

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (37 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an azido silyl enol ether (1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-10** (yield: 73%). ¹H NMR (400 MHz, Chloroform-*d*) δ 7.96 - 7.91 (m, 2H), 7.39 (d, *J =* 8.2 Hz, 2H), 5.15 (d, *J =* 8.4 Hz, 1H), 4.39 (s, 3H), 3.72 (s, 3H), 3.06 (qdd, *J =* 17.9, 8.1, 6.3 Hz, 2H), 2.34 - 2.23 (m, 1H), 2.05 (dq, *J =* 14.8, 7.8 Hz, 1H), 1.39 (s, 9H). ¹³C NMR (101 MHz, Chloroform-*d*) δ 198.17, 172.78, 155.38, 140.63, 136.45, 128.54, 128.10, 79.98, 54.18, 53.00, 52.37, 34.54, 28.22, 26.95.

### Example 21 Preparation of a compound AA-11 with an olefin EB-Br

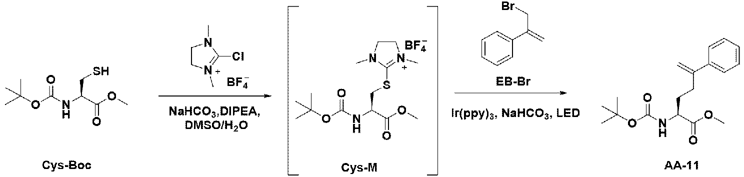

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an olefin **EB-Br** (98 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-11** (yield: 53%). ¹H NMR (400 MHz, Chloroform-*d*) δ 7.39-7.27 (m, 5H), 5.29 (s, 1H), 5.08 (d, *J* = 1.5 Hz, 1H), 5.02 (d, *J* = 9.5 Hz, 1H), 4.35 (s, 1H), 3.71 (s, 3H), 2.56 (t, *J* = 8.7 Hz, 2H), 1.98 (d, *J* = 13.8 Hz, 1H), 1.75 (dq, *J* = 14.8, 7.8 Hz, 1H), 1.40 (s, 9H).

### Example 22 Preparation of a compound AA-11 with an olefin EB-SO₂Ph

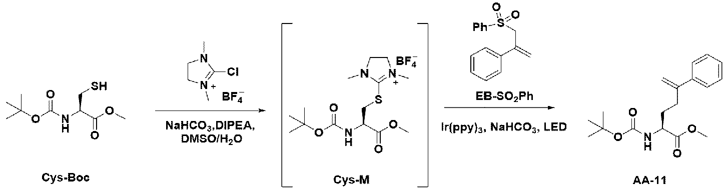

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an olefin **EB-SO₂Ph** (129 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-11** (yield: 52%).

### Example 23 Preparation of a compound AA-11 with EB-TMS

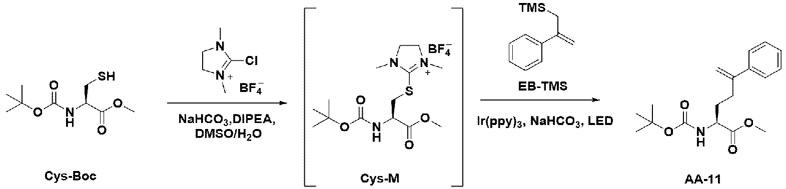

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and **EB-TMS** (95 mg, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-11** (yield: 62%).

### Example 24 Preparation of a compound AA-12

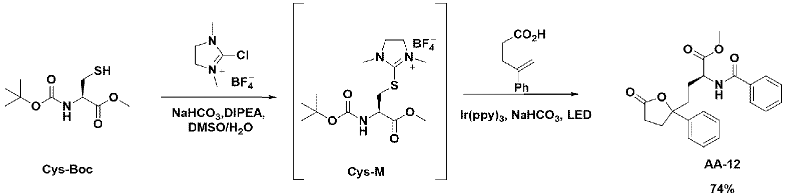

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an olefin (0.26 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-12** (yield: 74%). ¹H NMR (400 MHz, Chloroform-*d*) δ 7.82 - 7.74 (m, 2H), 7.55 - 7.49 (m, 1H), 7.47 - 7.41 (m, 2H), 7.35 (ttd, *J =* 7.0, 6.1, 1.4 Hz, 2H), 7.31 - 7.25 (m, 3H), 6.71 (dd, *J =* 40.1, 8.0 Hz, 1H), 4.75 (dtd, *J =* 21.7, 8.3, 4.3 Hz, 1H), 3.71 (d, *J =* 8.5 Hz, 3H), 2.65 - 2.36 (m, 4H), 2.24 - 1.98 (m, 3H), 1.89 - 1.66 (m, 1H). ¹³C NMR (176 MHz, Chloroform-*d*) δ 176.32, 172.63, 172.54, 167.18, 142.22, 141.92, 133.61, 133.60, 131.90, 128.77, 128.74, 128.63, 128.61, 127.84, 127.80, 127.09, 127.08, 124.51, 124.45, 88.66, 88.58, 52.60, 52.12, 52.03, 38.24, 38.03, 35.72, 35.60, 28.43, 28.40, 27.68, 27.41.

### Example 25 Preparation of a compound AA-13

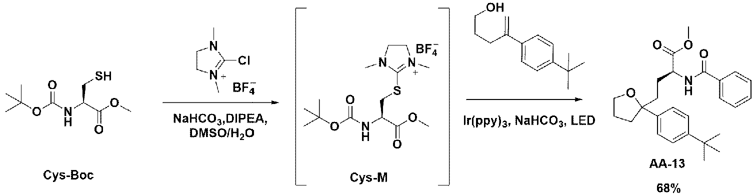

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an olefin (0.26 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-13** (yield: 68%). ¹H NMR (400 MHz, Chloroform-*d*) δ 7.85 - 7.79 (m, 2H), 7.54 - 7.48 (m, 1H), 7.48 - 7.40 (m, 2H), 7.37 - 7.28 (m, 2H), 7.27 - 7.17 (m, 2H), 4.65 (dtd, *J* = 31.0, 7.6, 4.5 Hz, 1H), 4.06 - 3.94 (m, 1H), 3.91 (td, *J =* 6.5, 6.0, 1.2 Hz, 1H), 3.70 (d, *J* = 10.7 Hz, 3H), 2.21 - 1.60 (m, 8H), 1.31 (d, *J* = 1.4 Hz, 9H). ¹³C NMR (176 MHz, Chloroform-*d*) δ 173.02, 172.93, 167.19, 167.16, 149.30, 149.29, 142.72, 142.69, 134.10, 133.98, 131.66, 131.61, 128.52, 128.50, 127.17, 127.14, 125.06, 124.83, 124.77, 86.41, 86.24, 67.91, 67.69, 52.96, 52.47, 52.32, 52.29, 39.14, 38.74, 37.80, 37.59, 34.37, 31.39, 27.13, 26.97, 25.55, 25.51.

### Example 26 Preparation of a compound AA-01 with a thiazolium salt for activation

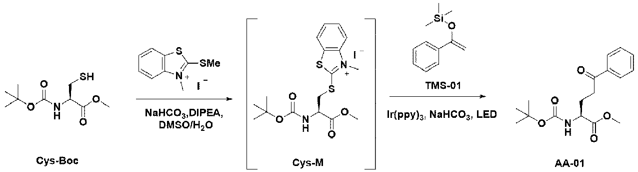

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then methyl thio ether thiazolium iodide (37 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-01** (yield: 88%).

### Example 27 Preparation of a compound AA-14 with phenylacetylene

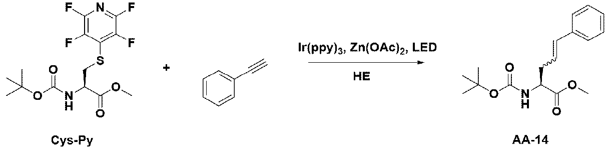

A compound **Cys-Py** (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), a Hantzsch ester (HE, 51.0 mg, 0.20 mmol), and phenylacetylene (55 µL, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-14** (yield: 65%, *E*/*Z* = 20:1). Methyl (E)-2-((tert-butoxycarbonyl)amino)-5-phenylpent-4-enoate ¹H NMR (400 MHz, Chloroform-d) δ 7.35-7.26 (m, 4H), 7.22 (d, J = 6.8 Hz, 1H), 6.44 (d, J = 15.7 Hz, 1H), 6.05 (dt, J = 15.2, 7.3 Hz, 1H), 5.06 (d, J = 8.7 Hz, 1H), 4.44 (m, 1H), 3.74 (s, 3H), 2.65 (m, 2H), 1.41 (s, 9H). Methyl (Z)-2-((tert-butoxycarbonyl)amino)-5-phenylpent-4-enoate ¹H NMR (400 MHz, Chloroform-d) δ 7.35-7.26 (m, 4H), 7.22 (d, J = 6.8 Hz, 1H), 6.58 (d, J = 11.7 Hz, 1H), 5.57 (dt, J = 11.6, 7.2 Hz, 1H), 5.04-4.99 (m, 1H), 4.33-4.26 (m, 1H), 3.74 (s, 3H), 2.90-2.79 (m, 2H), 1.40 (s, 9H).

### Example 28 Preparation of a compound AA-15

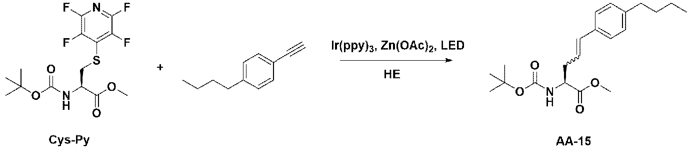

A compound **Cys-Py** (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), a Hantzsch ester (HE, 51.0 mg, 0.20 mmol), and an alkyne (55 µL, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-15** (yield: 54%, *E*/*Z =* 20:1). ¹H NMR (400 MHz, Chloroform-*d*) δ 7.23 (d, *J* = 8.2 Hz, 2H), 7.09 (d, *J =* 7.9 Hz, 2H), 6.41 (d, *J =* 15.8 Hz, 1H), 5.98 (dt, *J =* 15.4, 7.4 Hz, 1H), 5.05 (d, *J =* 8.3 Hz, 1H), 4.47 - 4.38 (m, 1H), 3.73 (s, 3H), 2.65 (dq, *J =* 13.9, 8.1, 6.9 Hz, 2H), 2.56 (t, *J =* 7.7 Hz, 2H), 1.57 - 1.50 (m, 2H), 1.41 (s, 9H), 1.36 - 1.29 (m, 2H), 0.90 (t, *J =* 7.3 Hz, 3H). ¹³C NMR (101 MHz, CDCl₃) δ 172.60, 155.24, 142.49, 134.32, 133.94, 128.61, 126.19, 122.60, 79.98, 53.24, 52.32, 36.14, 35.35, 33.59, 28.31, 22.33, 13.94.

### Example 29 Preparation of a compound AA-16 with an olefin

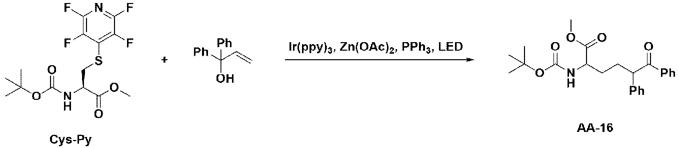

A compound **Cys-Py** (77.0 mg, 0.20 mmol) was dissolved in 2 mL of DMSO, and anhydrous Zn(OAc)₂ (22.0 mg, 0.12 mmol), Ir(ppy)₃ (2.0 mg, 0.003 mmol), 1,1-diphenylpropenol (63.0 mg, 0.30 mmol), and triphenylphosphine (10.5 mg, 0.04 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **AA-16** (66.0 mg, yield: 81%, dr: 1:1).¹H NMR (400 MHz, CDCl₃) δ 7.94 (d, J = 7.0 Hz, 2H), 7.52-7.43 (m, 1H), 7.37 (t, J = 7.0 Hz, 2H), 7.32-7.25 (m, 4H), 7.24-7.15 (m, 1H), 5.07 (dd, J = 18.2, 7.4 Hz, 1H), 4.65-4.50 (m, 1H), 4.33 (s, 1H), 3.69 (d, J = 12.9 Hz, 3H), 2.32-2.14 (m, 1H), 1.98 - 1.80 (m, 2H), 1.70 - 1.52 (m, 1H), 1.43 (d, J = 3.4 Hz, 9H). ¹³C NMR (101 MHz, CDCl₃) δ 199.42, 173.21, 155.52, 139.32, 138.95, 136.76, 136.68, 133.08, 133.05, 129.15, 128.81, 128.65, 128.63, 128.34, 128.20, 127.36, 127.32, 80.05, 80.01, 53.23, 52.92, 52.40, 52.34, 30.76, 30.30, 29.76, 29.35, 28.41. HRMS (ESI) calcd for C₂₄H₂₉NNaO₅ [M + Na] + m/z = 434.1938, found: 434.1943.

This method was used for the modification of a cysteine-containing polypeptide, as shown in the following examples:

### Example 30 Preparation of a polypeptide compound DP-01

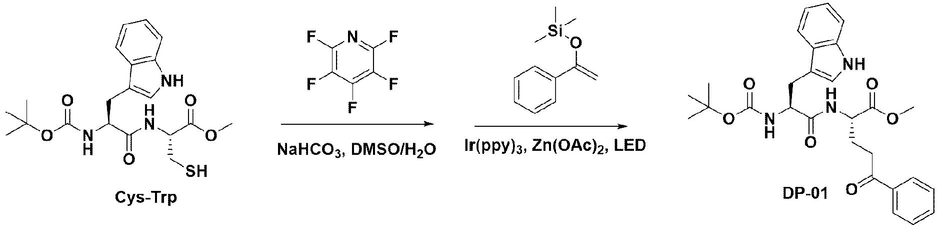

A compound **Cys-Trp** (84.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-01** (67 mg, yield: 66%). ¹H NMR (400 MHz, Chloroform-*d*) δ 8.44 (d, *J =* 43.7 Hz, 1H), 7.84 (d, *J =* 7.7 Hz, 2H), 7.60 (d, *J =* 8.0 Hz, 1H), 7.55 (t, *J =* 7.4 Hz, 1H), 7.42 (t, *J =* 7.6 Hz, 2H), 7.36 - 7.19 (m, 1H), 7.10 (ddd, *J =* 25.5, 17.9, 11.0 Hz, 3H), 6.72 - 6.41 (m, 1H), 5.19 (s, 1H), 4.54 (td, *J* = 7.6, 5.2 Hz, 1H), 4.46 (t, *J =* 7.0 Hz, 1H), 3.63 (d, *J* = 3.3 Hz, 3H), 3.29 (dd, *J* = 14.5, 5.7 Hz, 1H), 3.16 (dd, *J* = 14.6, 6.8 Hz, 1H), 2.95-2.71 (m, 2H), 2.30-2.13 (m, 1H), 1.99 (tdd, *J* = 14.4, 8.6, 4.7 Hz, 1H), 1.41 (d, *J* = 4.8 Hz, 9H). ¹³C NMR (101 MHz, Chloroform-*d*) δ 199.01, 172.81, 171.87, 155.42, 136.51, 136.21, 133.17, 128.54, 128.01, 127.46, 123.33, 122.09, 119.60, 118.78, 118.67, 111.23, 110.22, 80.13, 55.42, 52.35, 51.90, 48.09, 34.11, 28.23, 26.43, 18.22. It should be noted that hydrogen and carbon nuclear magnetic resonance spectra for the product had only one set of peaks, which also proved that a product with a single configuration, rather than a product with a racemic stereo center, was produced.

### Example 31 Preparation of a polypeptide compound DP-02

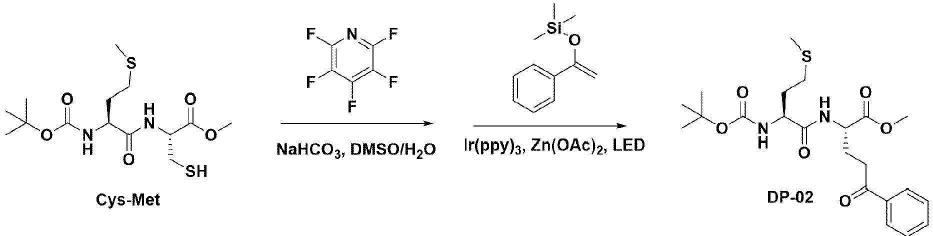

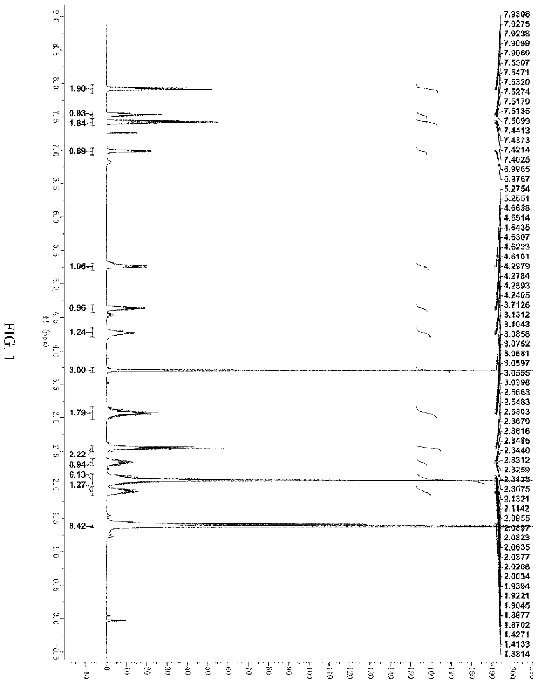

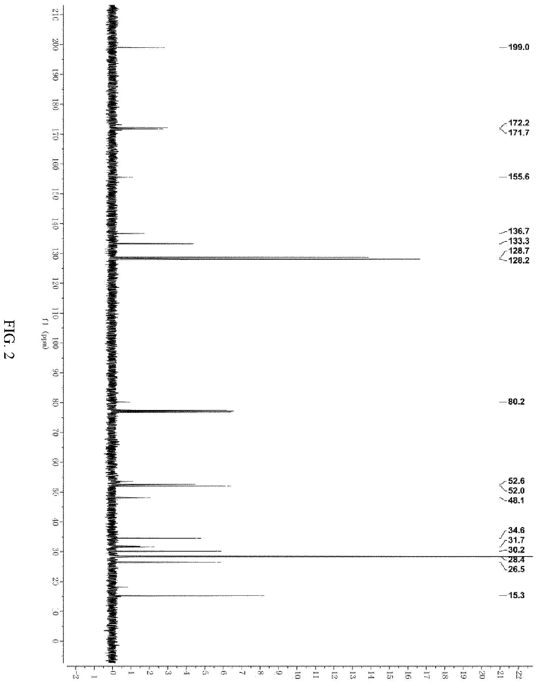

A compound **Cys-Met** (73.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.1 mL, 0.5 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-02** (85 mg, yield: 94%). Hydrogen and carbon nuclear magnetic resonance spectra for the product were shown in FIG. 1 and FIG. 2, respectively. ¹H NMR (400 MHz, Chloroform-*d*) δ 7.98-7.85 (m, 2H), 7.58-7.48 (m, 1H), 7.42 (t, *J =* 7.7 Hz, 2H), 6.99 (d, *J* = 7.9 Hz, 1H), 5.27 (d, *J =* 8.1 Hz, 1H), 4.64 (td, *J =* 8.2, 5.1 Hz, 1H), 4.27 (q, *J =* 7.7 Hz, 1H), 3.71 (s, 3H), 3.07 (tdd, *J =* 11.9, 9.3, 4.4 Hz, 2H), 2.55 (t, *J =* 7.2 Hz, 2H), 2.34 (dtd, *J =* 14.5, 7.2, 5.1 Hz, 1H), 2.07 (d, *J =* 7.5 Hz, 5H), 1.90 (dq, *J =* 14.7, 7.3 Hz, 1H), 1.38 (s, 9H). ¹³C NMR (101 MHz, Chloroform-*d*) δ 198.89, 172.05, 171.59, 155.44, 136.53, 133.18, 128.56, 128.01, 80.03, 52.42, 51.87, 48.00, 34.43, 31.51, 30.01, 28.21, 26.33, 15.13.

### Example 32 Preparation of a polypeptide compound DP-03

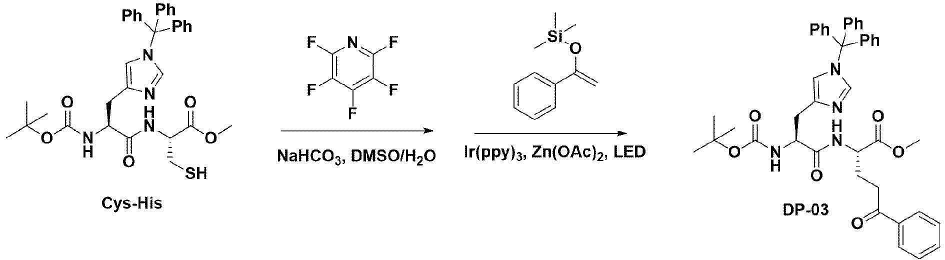

A compound **Cys-His** (123.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-03** (118 mg, yield: 84%). ¹H NMR (400 MHz, Chloroform-*d*) δ 7.87 (d, *J =* 7.7 Hz, 2H), 7.61 (s, 1H), 7.48 (t, *J =* 7.4 Hz, 1H), 7.35 (t, *J =* 7.7 Hz, 2H), 7.27 (dd, *J* = 5.2, 1.9 Hz, 10H), 7.12-6.95 (m, 6H), 6.62 (s, 1H), 6.09 (s, 1H), 4.58 (td, *J* = 7.9, 5.3 Hz, 1H), 4.40 (s, 1H), 3.62 (s, 3H), 3.01 (dddd, *J =* 47.8, 18.0, 8.7, 6.0 Hz, 4H), 2.44 (s, 1H), 2.34-2.19 (m, 1H), 2.04 (tt, *J =* 14.4, 6.9 Hz, 1H), 1.36 (s, 9H). ¹³C NMR (101 MHz, Chloroform-*d*) δ 199.55, 172.91, 172.22, 156.31, 142.29, 139.66, 136.76, 133.14, 129.80, 128.63, 128.18, 128.13, 119.75, 80.02, 54.73, 52.40, 51.53, 34.54, 30.33, 28.41, 25.73.

### Example 33 Preparation of a polypeptide compound DP-04

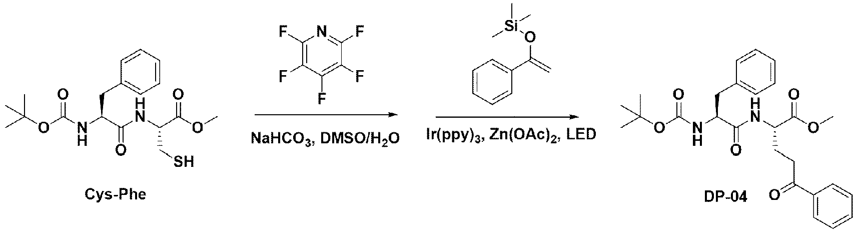

A compound **Cys-Phe** (77.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-04** (89 mg, yield: 95%). ¹H NMR (400 MHz, Chloroform-*d*) δ 7.90-7.86 (m, 2H), 7.55-7.49 (m, 1H), 7.41 (t, *J* = 7.7 Hz, 2H), 7.21-7.10 (m, 5H), 6.72 (d, *J=* 7.7 Hz, 1H), 5.03 (d, *J =* 7.5 Hz, 1H), 4.58 (td, *J =* 7.9, 5.2 Hz, 1H), 4.34 (d, *J =* 8.3 Hz, 1H), 3.66 (s, 3H), 3.05-2.83 (m, 4H), 2.30-2.19 (m, 1H), 2.06 (q, *J =* 7.5 Hz, 1H), 1.34 (s, 9H). ¹³C NMR (101 MHz, Chloroform-*d*) δ 198.82, 171.88, 171.28, 155.28, 136.54, 136.46, 133.14, 129.25, 128.52, 128.33, 128.00, 126.83, 80.06, 55.78, 52.37, 51.85, 38.09, 34.29, 28.17, 26.50.

### Example 34 Preparation of a polypeptide compound DP-05

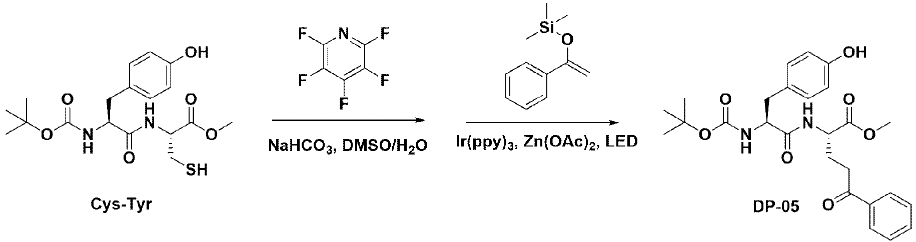

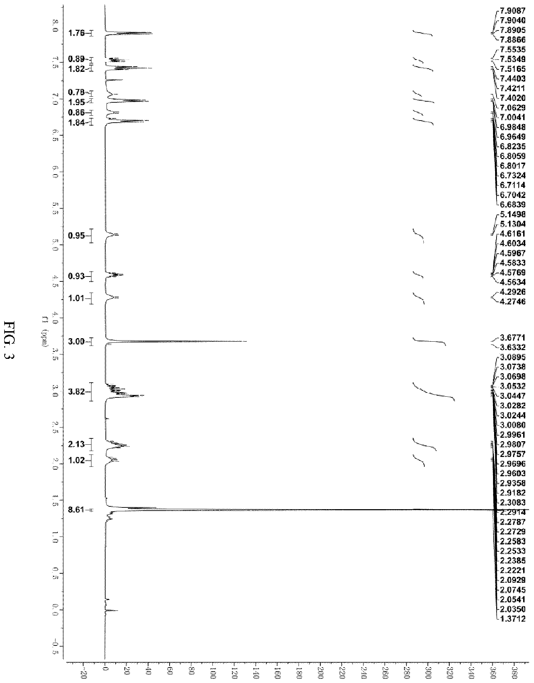

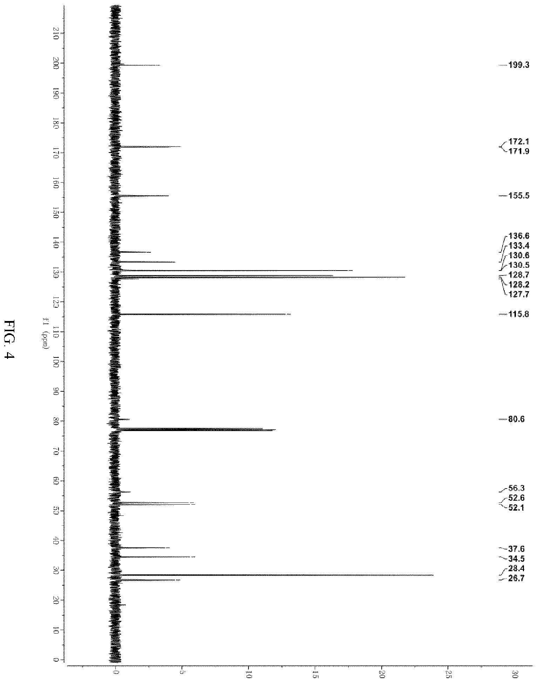

A compound Cys-Tyr (84.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-05** (79 mg, yield: 81%). Hydrogen and carbon nuclear magnetic resonance spectra for the product were shown in FIG. 3 and FIG. 4, respectively. ¹H NMR (400 MHz, Chloroform-*d*) δ 7.94-7.86 (m, 2H), 7.53 (t, *J =* 7.4 Hz, 1H), 7.42 (t, *J =* 7.7 Hz, 2H), 7.06 (s, 1H), 6.97 (d, *J =* 8.0 Hz, 2H), 6.85-6.77 (m, 1H), 6.71 (dd, *J =* 11.1, 8.2 Hz, 2H), 5.14 (d, *J =* 7.8 Hz, 1H), 4.59 (td, *J =* 7.9, 5.3 Hz, 1H), 4.34-4.19 (m, 1H), 3.68 (s, 3H), 3.11- 2.86 (m, 4H), 2.35-2.18 (m, 2H), 2.05 (t, *J =* 7.9 Hz, 1H), 1.37 (s, 9H). ¹³C NMR (101 MHz, Chloroform-*d*) δ 199.32, 172.05, 171.89, 155.50, 136.64, 133.40, 130.55, 130.48, 128.75, 128.20, 127.72, 115.77, 80.57, 56.26, 52.64, 52.08, 37.55, 34.50, 28.35, 26.71.

### Example 35 Preparation of a polypeptide compound DP-06

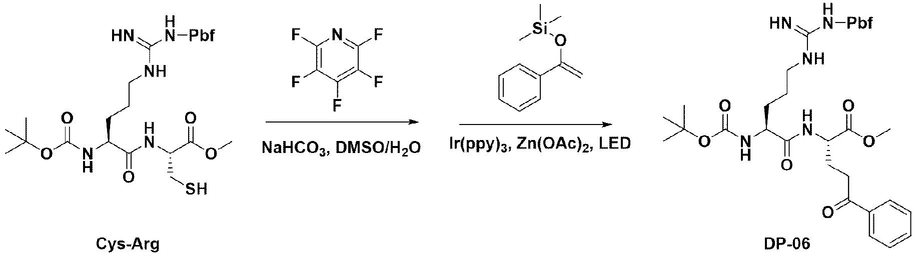

A compound **Cys-Arg** (129.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-06** (131 mg, yield: 90%). ¹H NMR (400 MHz, Chloroform-*d*) δ 7.90-7.82 (m, 2H), 7.57 (d, *J* = 7.7 Hz, 1H), 7.47 (td, *J* = 7.2, 1.8 Hz, 1H), 7.35 (td, *J* = 7.9, 2.1 Hz, 2H), 6.31 (s, 2H), 6.20 (s, 1H), 5.61 (dd, *J =* 6.9, 2.9 Hz, 1H), 4.54 (td, *J =* 8.2, 5.2 Hz, 1H), 4.18 (s, 1H), 3.64 (d, *J* = 2.3 Hz, 3H), 3.17 (d, *J =* 19.2 Hz, 2H), 3.08 -2.98 (m, 2H), 2.88 (s, 2H), 2.80-2.64 (m, 1H), 2.56 (d, *J* = 2.3 Hz, 6H), 2.51 (s, 3H), 2.44 (s, 3H), 2.33-2.22 (m, 1H), 2.07 (dd, *J* = 15.0, 8.0 Hz, 1H), 2.01 (s, 3H), 1.88-1.73 (m, 1H), 1.55 (h, *J* = 9.2, 8.4 Hz, 3H), 1.40 (d, *J* = 1.8 Hz, 6H), 1.34 (d, *J =* 2.0 Hz, 9H). ¹³C NMR (101 MHz, Chloroform-*d*) δ 199.17, 172.82, 172.45, 158.67, 156.46, 155.81, 138.27, 136.60, 133.12, 133.00, 132.21, 128.54, 128.05, 124.56, 117.41, 86.32, 79.75, 52.39, 51.99, 43.21, 40.81, 34.57, 29.83, 28.56, 28.29, 25.82, 25.34, 19.26, 17.92, 12.41.

### Example 36 Preparation of a polypeptide compound DP-07

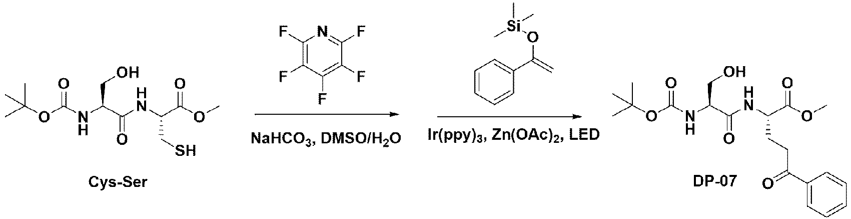

A compound **Cys-Ser** (65.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-07** (65 mg, yield: 80%). ¹H NMR (400 MHz, Chloroform-*d*) δ 7.94-7.77 (m, 2H), 7.54-7.49 (m, 1H), 7.41 (dd, *J =* 8.4, 7.0 Hz, 2H), 7.37-7.33 (m, 1H), 5.60 (d, *J* = 7.5 Hz, 1H), 4.62 (td, *J =* 8.2, 4.9 Hz, 1H), 4.17 (s, 1H), 3.96 (d, *J =* 11.1 Hz, 1H), 3.70 (s, 3H), 3.63 (s, 1H), 3.51 (s, 1H), 3.05 (dt, *J =* 7.0, 3.4 Hz, 2H), 2.95 (s, 1H), 2.33 (dtd, *J* = 14.3, 7.2, 5.0 Hz, 1H), 2.11 (ddd, *J =* 13.7, 8.5, 6.9 Hz, 1H), 1.37 (s, 9H). ¹³C NMR (101 MHz, Chloroform-*d*) δ 199.04, 172.32, 171.36, 155.86, 136.44, 133.27, 128.59, 128.01, 80.34, 62.91, 55.30, 52.61, 52.07, 42.58, 34.45, 28.20, 26.07.

### Example 37 Preparation of a polypeptide compound DP-07

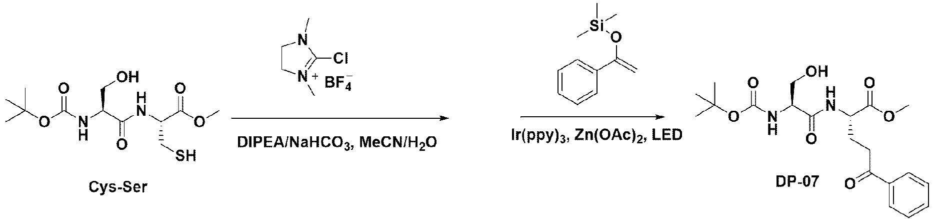

A compound **Cys-Boc** (52.0 mg, 0.2 mmol) was dissolved in 4 mL of CH₃CN, 0.1 mL of water was added, then NaHCO₃ (18.0 mg, 0.21 mmol) and DIPEA (38 µL, 0.21 mmol) were added, and then 2-chloro-1,3-dimethylimidazolium tetrafluoroborate (48 mg, 0.22 mmol) was added. Stirring was conducted at room temperature to allow a reaction for 60 min. Anhydrous NaHCO₃ (18.0 mg, 0.21 mmol), Ir(ppy)₃ (2.0 mg, 0.003 mmol), and an olefin (0.26 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-07** (56 mg, yield: 70%). ¹H NMR (400 MHz, Chloroform-*d*) δ 7.94-7.77 (m, 2H), 7.54-7.49 (m, 1H), 7.41 (dd, *J* = 8.4, 7.0 Hz, 2H), 7.37-7.33 (m, 1H), 5.60 (d, *J* = 7.5 Hz, 1H), 4.62 (td, *J =* 8.2, 4.9 Hz, 1H), 4.17 (s, 1H), 3.96 (d, *J =* 11.1 Hz, 1H), 3.70 (s, 3H), 3.63 (s, 1H), 3.51 (s, 1H), 3.05 (dt, *J* = 7.0, 3.4 Hz, 2H), 2.95 (s, 1H), 2.33 (dtd, *J* = 14.3, 7.2, 5.0 Hz, 1H), 2.11 (ddd, *J =* 13.7, 8.5, 6.9 Hz, 1H), 1.37 (s, 9H). ¹³C NMR (101 MHz, Chloroform-d) δ 199.04, 172.32, 171.36, 155.86, 136.44, 133.27, 128.59, 128.01, 80.34, 62.91, 55.30, 52.61, 52.07, 42.58, 34.45, 28.20, 26.07.

### Example 38 Preparation of a polypeptide compound DP-08

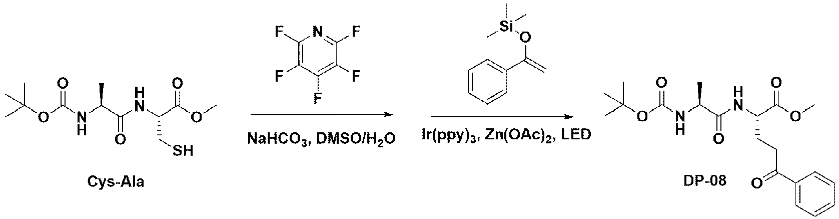

A compound **Cys-Ala** (61.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-08** (75 mg, yield: 95%). ¹H NMR (400 MHz, Chloroform-*d*) δ 7.91-7.86 (m, 2H), 7.52-7.46 (m, 1H), 7.39 (t, *J* = 7.6 Hz, 2H), 6.96 (d, *J* = 8.0 Hz, 1H), 5.14 (d, *J =* 7.6 Hz, 1H), 4.61 (td, *J =* 8.2, 5.1 Hz, 1H), 4.18-4.09 (m, 1H), 3.68 (s, 3H), 3.14-2.92 (m, 2H), 2.34- 2.23 (m, 1H), 2.07 (dtd, *J =* 14.3, 8.2, 6.1 Hz, 1H), 1.35 (s, 9H), 1.28 (d, *J =* 7.0 Hz, 3H). ¹³C NMR (101 MHz, Chloroform-*d*) δ 198.96, 172.83, 172.19, 155.37, 136.52, 133.12, 128.51, 127.97, 79.89, 52.36, 51.74, 50.11, 47.92, 34.43, 28.19, 26.49, 18.12.

### Example 39 Preparation of a polypeptide compound DP-09

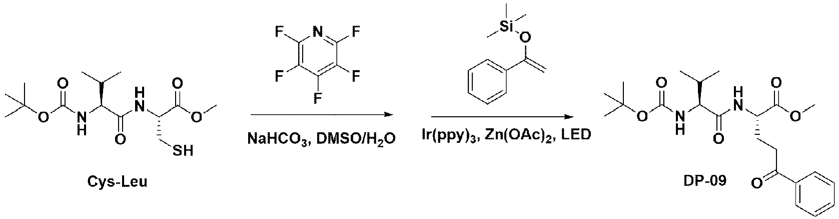

A compound **Cys-Leu** (67.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-09** (82 mg, yield: 98%). ¹H NMR (400 MHz, Chloroform-*d*) δ 7.90-7.85 (m, 2H), 7.52-7.45 (m, 1H), 7.38 (t, *J* = 7.6 Hz, 2H), 6.87 (d, *J* = 7.7 Hz, 1H), 5.15 (d, *J* = 8.7 Hz, 1H), 4.60 (td, *J =* 8.1, 5.1 Hz, 1H), 3.94-3.85 (m, 1H), 3.67 (s, 3H), 3.04 (qdd, *J =* 18.1, 8.0, 6.4 Hz, 2H), 2.33-2.18 (m, 1H), 2.13-1.95 (m, 2H), 1.34 (s, 9H), 0.88 (dd, *J* = 17.8, 6.8 Hz, 6H). ¹³C NMR (101 MHz, Chloroform-*d*) δ 199.01, 172.21, 171.75, 155.77, 136.45, 133.19, 128.54, 127.99, 79.68, 59.92, 52.41, 51.82, 34.51, 30.86, 28.22, 26.27, 19.12, 17.84.

### Example 40 Preparation of a polypeptide compound DP-10

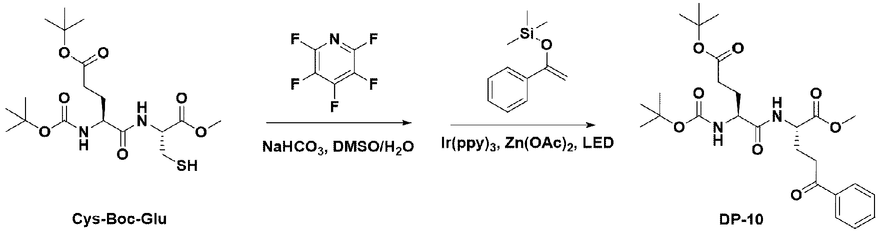

A compound **Cys-Boc-Glu** (84.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-10** (90 mg, yield: 89%). ¹H NMR (400 MHz, Chloroform-*d*) δ 7.89 (dd, *J* = 8.2, 1.5 Hz, 2H), 7.52-7.47 (m, 1H), 7.39 (t, *J* = 7.6 Hz, 2H), 7.06 (d, *J* = 7.9 Hz, 1H), 5.30 (d, *J =* 7.8 Hz, 1H), 4.60 (td, *J =* 8.1, 5.1 Hz, 1H), 4.11 (d, *J =* 7.1 Hz, 1H), 3.68 (s, 3H), 3.04 (qdd, *J =* 17.9, 8.2, 6.2 Hz, 2H), 2.37- 2.26 (m, 3H), 2.12-1.98 (m, 2H), 1.85 (dt, *J =* 14.4, 7.5 Hz, 1H), 1.38 (s, 9H), 1.35 (s, 9H). ¹³C NMR (101 MHz, Chloroform-*d*) δ 198.81, 172.67, 172.08, 171.73, 155.56, 136.51, 133.14, 128.53, 128.00, 80.83, 79.89, 53.93, 52.40, 51.78, 34.39, 31.64, 28.20, 28.00, 27.61, 26.39.

### Example 41 Preparation of a polypeptide compound DP-11

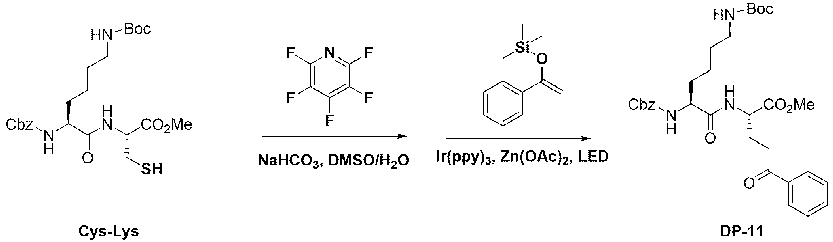

A compound **Cys-Lys** (99.4 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-11** (105 mg, yield: 90%). ¹H NMR (400 MHz, Chloroform-*d*) δ 7.92 (d, *J =* 7.7 Hz, 2H), 7.53 (t, *J* = 7.3 Hz, 1H), 7.42 (t, *J =* 7.6 Hz, 2H), 7.35 - 7.22 (m, 5H), 7.01 (d, *J =* 7.8 Hz, 1H), 5.58 (d, *J* = 7.8 Hz, 1H), 5.11 - 4.96 (m, 2H), 4.79 - 4.55 (m, 2H), 4.18 (q, *J* = 7.3 Hz, 1H), 3.72 (s, 3H), 3.07 (tq, *J =* 15.8, 8.3, 5.8 Hz, 4H), 2.34 (dq, *J =* 13.3, 6.7 Hz, 1H), 2.13 (dp, *J =* 14.7, 7.5 Hz, 1H), 1.82 (dq, *J =* 13.8, 7.0 Hz, 1H), 1.63 (dt, *J* = 14.4, 7.3 Hz, 1H), 1.48 - 1.28 (m, 4H) 1.40 (s, 9H). ¹³C NMR (101 MHz, Chloroform-*d*) δ 199.16, 172.29, 172.02, 156.22, 136.50, 136.22, 133.29, 128.62, 128.48, 128.12, 128.07, 128.00, 79.08, 66.93, 54.75, 52.52, 51.96, 45.04, 39.80, 34.58, 29.46, 28.42, 26.13, 22.27. [α]_{D}¹⁵ = 0 (c = 0.23, CH₂Cl₂). ESI-HRMS (m/z): [M+Na]⁺ Calcd. for [C₃₁H₄₁N₃NaO₈]⁺: 606.2786, found 606.2783.

### Example 42 Preparation of a polypeptide compound DP-12

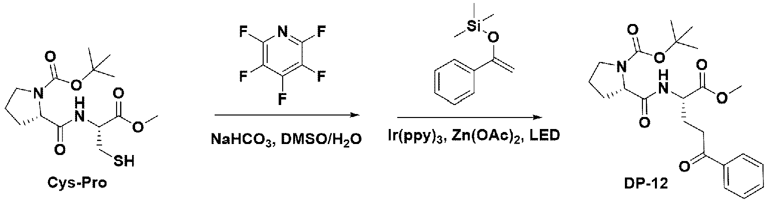

A compound **Cys-Pro** (67.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-12** (73 mg, yield: 87%). ¹H NMR (400 MHz, Chloroform-*d*) δ 7.92-7.84 (m, 2H), 7.49 (t, *J =* 7.5 Hz, 1H), 7.38 (t, *J =* 7.6 Hz, 2H), 6.84 (s, 1H), 4.65-4.54 (m, 1H), 4.19 (s, 1H), 3.70 -3.63 (m, 3H), 3.33 (d, *J =* 38.1 Hz, 2H), 3.02 (dt, *J =* 15.4, 7.0 Hz, 2H), 2.37-1.92 (m, 5H), 1.82 (d, *J =* 42.6 Hz, 3H), 1.36 (s, 9H).¹³C NMR (101 MHz, Chloroform-*d*) δ 198.77, 172.12, 136.54, 133.09, 128.50, 127.95, 80.28, 59.96, 52.31, 51.72, 46.98, 34.41, 28.22, 26.61, 24.47, 23.64.

### Example 43 Preparation of a polypeptide compound DP-13

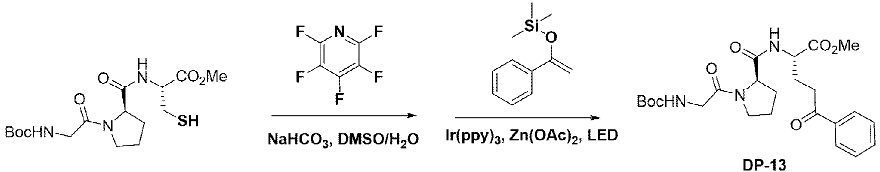

A compound **Cys-Pro-Gly** (67.0 mg, 0.20 mmol) was dissolved in 4 mL of DMSO, 0.1 mL of water was added, then NaHCO₃ (18.0 mg, 0.21 mmol) was added, and then 25 µ L of pentafluoropyridine was added. Stirring was conducted at room temperature to allow a reaction for 20 min. To a reaction solution, 6 mL of DMSO was added, and anhydrous Zn(OAc)₂ (22.0 mg, 0.12 mmol), Ir(ppy)₃ (2.0 mg, 0.003 mmol), and an silyl enol ether **TMS-01** (0.2 mL, 1.0 mmol) were added. Nitrogen replacement was conducted 3 times. Stirring was conducted for 14 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-13** (73 mg, yield: 75%). ¹H NMR (400 MHz, Chloroform-*d*) δ 7.97 (d, *J =* 7.4 Hz, 2H), 7.55 (t, *J =* 7.3 Hz, 1H), 7.47 (t, *J =* 7.6 Hz, 2H), 7.14 (d, *J =* 8.1 Hz, 1H), 5.40 (s, 1H), 4.66 (td, *J =* 8.6, 4.8 Hz, 1H), 4.52 - 4.46 (m, 1H), 4.00 (dd, *J =* 17.4, 5.1 Hz, 1H), 3.84 (dd, *J =* 17.3, 3.9 Hz, 1H), 3.76 (s, 3H), 3.48 - 3.32 (m, 2H), 3.11 (td, *J =* 7.1, 2.4 Hz, 2H), 2.43 - 2.34 (m, 1H), 2.29 - 2.19 (m, 1H), 2.14 - 1.91 (m, 4H), 1.45 (s, 9H). ¹³C NMR (101 MHz, Chloroform-*d*) δ 199.23, 172.38, 171.05, 168.30, 155.71, 136.69, 133.16, 128.60, 128.11, 79.72, 60.35, 60.26, 52.52, 51.86, 46.29, 43.11, 34.44, 28.36, 28.00, 26.43, 24.87. [α]_{D}¹⁵ = -42.2 (c = 0.66, CH₂Cl₂). ESI-HRMS calcd for C₂₄H₃₃N₃O₇Na⁺ [M+Na]⁺ m/z = 498.2211, found: 498.2208.

### Example 44 Preparation of a polypeptide compound DP-14

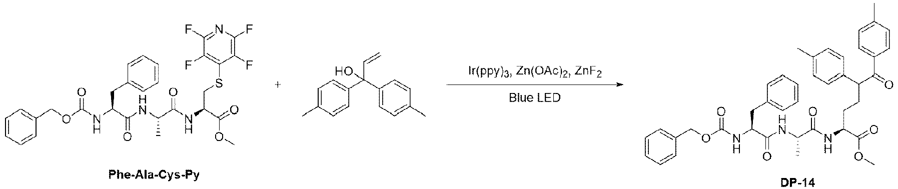

A compound **Phe-Ala-Cys-Py** (159 mg, 0.25 mmol) was dissolved in 5 mL of DMSO, and anhydrous 1,1-bis(4-methylphenyl)prop-2-en-1-ol (mg, 0.50 mmol), Zn(OAc)₂ (28 mg, 0.15 mmol), ZnF₂ (26 mg, 0.25 mmol), and Ir(ppy)₃ (3 mg, 0.005 mmol) were added. Nitrogen replacement was conducted 3 times. Stirring was conducted for 15 h under the irradiation of blue LED. At the end of a reaction, a reaction solution was poured into 20 mL of water, and extraction was conducted with ethyl acetate (20 mL × 3). Organic phases were combined, dried with anhydrous sodium sulfate, filtered, and spin-dried for removing the solvent to produce a crude product. The crude product was subjected to column chromatography to produce a target product **DP-14** (100 mg, yield: 58%).

methyl(5*S*,8*S*,11*S*)-5-benzyl-8-methyl-3,6,9-trioxo-11-(4-oxo-3,4-di-*p*-tolylbutyl)-1-phenyl-2-oxa-4,7,10-triazadodecan-12-oate:(dr 1:1) ¹H NMR (700 MHz, CDCl₃) δ 7.82 (t, *J =* 7.9 Hz, 2H), 7.35 - 7.29 (m, 3H), 7.28 - 7.26 (m, 2H), 7.23 - 7.19 (m, 2H), 7.18 - 7.10 (m, 7H), 7.03 (dd, *J* = 10.0, 7.9 Hz, 2H), 6.99 - 6.93 (m, 1H), 6.77 (dd, *J =* 18.8, 7.2 Hz, 1H), 5.55 (t, *J =* 6.9 Hz, 1H), 5.08 - 4.97 (m, 2H), 4.60 - 4.44 (m, 4H), 3.68 (s, 1.5H), 3.66 (s, 1.5H), 3.02 (dd, *J* = 13.7, 6.8 Hz, 2H), 2.32 (s, 1.5H), 2.31 (s, 1.5H), 2.22 (s, 1.5H), 2.20 (s, 1.5H), 2.19 - 2.12 (m, 1H), 1.87 - 1.74 (m, 2H), 1.71 - 1.61 (m, 1H), 1.35 - 1.28 (m, 3H). ¹³C NMR (176 MHz, CDCl₃) δ 199.2, 199.1, 172.5, 172.4, 171.8, 171.8, 171.0, 170.9, 156.2, 143.9, 143.9, 136.9, 136.9, 136.4, 136.4, 136.3, 136.3, 136.1, 134.1, 134.0, 129.9, 129.8, 129.4, 129.3, 129.3, 129.0, 128.7, 128.7, 128.6, 128.2, 128.1, 128.1, 127.9, 127.1, 127.1, 67.1, 56.2, 52.5, 52.5, 52.4, 52.4, 52.2, 52.2, 49.1, 49.0, 38.5, 38.5, 30.4, 29.9, 29.8, 29.2, 21.7, 21.7, 21.1, 21.0, 18.5, 18.4.

### Example 45 Preparation of a polypeptide compound DP-15

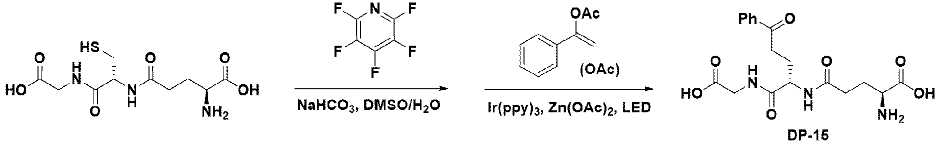

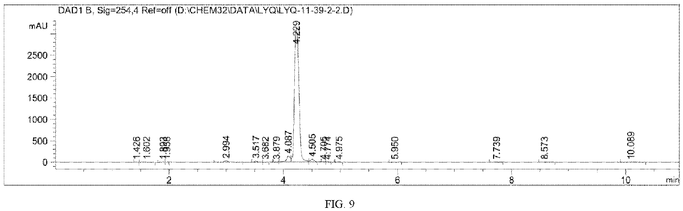

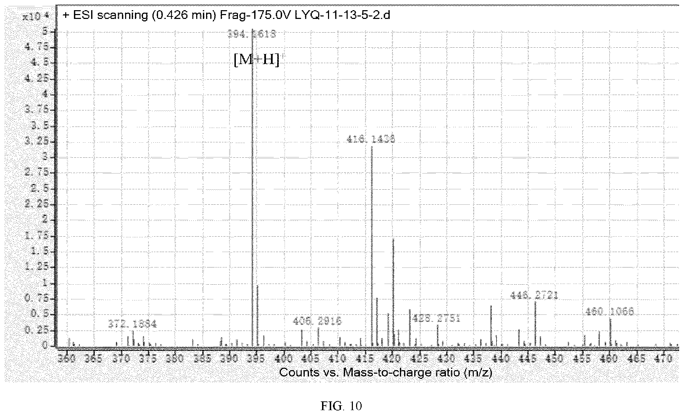

Glutathione (2.0 mg) was dissolved in 2 mL of DMSO, then 0.05 mL of phosphate-buffered saline (0.2 M, pH = 8) was added, then an equal equivalent of NaHCO₃ was added, and then 1.1 equivalent of pentafluoropyridine (1 mM in DMSO) was added. Stirring was conducted at room temperature to allow a reaction for 20 min. Anhydrous Zn(OAc)₂ (0.6 equivalent), Ir(ppy)₃ (0.1 equivalent), and an enol ester OAc (5 equivalent) were added to a reaction solution. Nitrogen replacement was conducted 3 times. Stirring was conducted for 14 h under the irradiation of blue LED (100 W, 5 cm away from the bottle). At the end of a reaction, filtration was conducted with a 0.22 µm filter cartridge. A product was then separated through semi-preparative liquid chromatography by SHIMADZU LC-20A HPL, with a separation yield of 82%. A separation column was an Agilent 7 µm (C-18) preparative column, a flow rate was 4 mL/min, and mobile phases were 0.1% trifluoroacetic acid (TFA) in water (solvent A) and 0.1% TAF in acetonitrile (solvent B). HPLC and mass spectrometry spectra of the polypeptide compound DP-15 were shown in FIG. 9 and FIG. 10, respectively.

¹H NMR (400 MHz, D₂O) δ 7.81 (d, *J =* 7.8 Hz, 2H), 7.52 (t, *J =* 7.6 Hz, 1H), 7.38 (t, *J* = 7.6 Hz, 2H), 4.29 - 4.24 (m, 1H), 3.81 (d, *J=* 4.3 Hz, 2H), 3.71 (t, *J* = 6.4 Hz, 1H), 3.08 (q, *J=* 6.7 Hz, 2H), 2.31 (t, *J =* 7.6 Hz, 2H), 2.14 - 2.05 (m, 1H), 2.00 - 1.91 (m, 3H). ¹³C NMR (101 MHz, D₂O) δ 203.61, 174.41, 174.06, 173.02, 172.68 (q, ²*J_{CF} =* 36 Hz, TFA), 136.11, 134.05, 128.83, 128.11, 53.13, 53.09, 41.07, 38.68, 34.32, 31.00, 25.69. HRMS (ESI) calcd for C₁₈H₂₄N₃O₇ [M + H] ⁺ m/z = 394.1609, found: 394.1618.

### Example 46 Preparation of a polypeptide compound DP-16

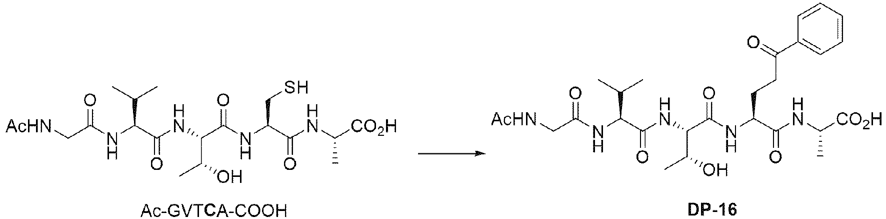

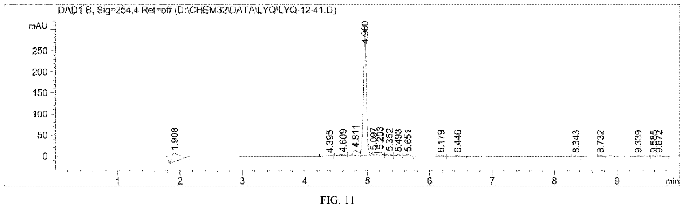

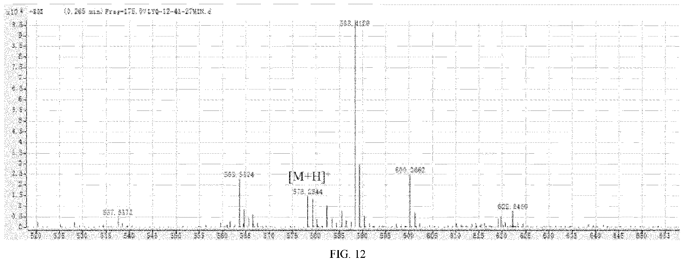

The polypeptide compound **DP-16** was prepared with reference to Example 44, and a separation yield was 69%. HPLC and mass spectrometry spectra of the polypeptide compound DP-16 were shown in FIG. 11 and FIG. 12, respectively. Analytical gradient 10-100% B over 10 min, 254 nm. Calculated Mass [M+H]⁺: 578.2821, Observed Mass [M+H]⁺: 578.2844

### Example 47 Preparation of a polypeptide compound DP-17

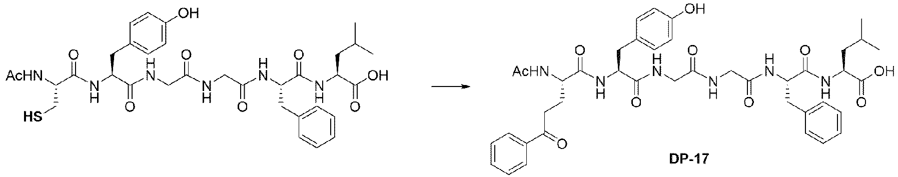

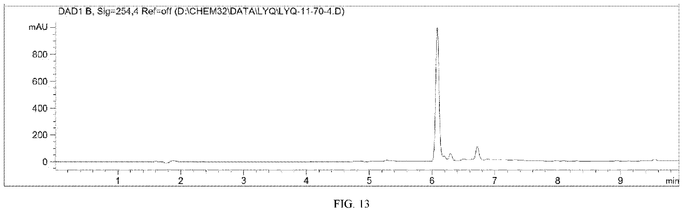

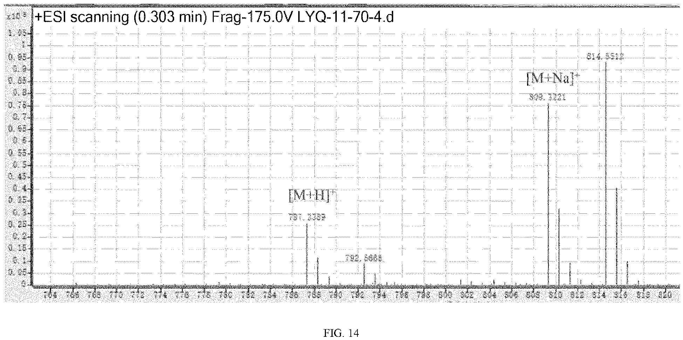

The polypeptide compound **DP-17** was prepared with reference to Example 44, and a separation yield was 57%. HPLC and mass spectrometry spectra of the polypeptide compound DP-17 were shown in FIG. 13 and FIG. 14, respectively. Analytical gradient 10-100% B over 10 min, 254 nm. Calculated Mass [M+H]⁺: 787.3661, Observed Mass [M+H]⁺: 787.3389.

### Example 48 Preparation of a polypeptide compound DP-18

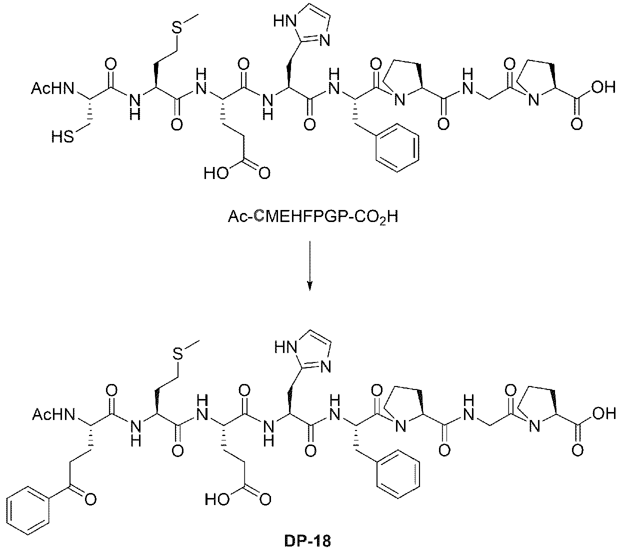

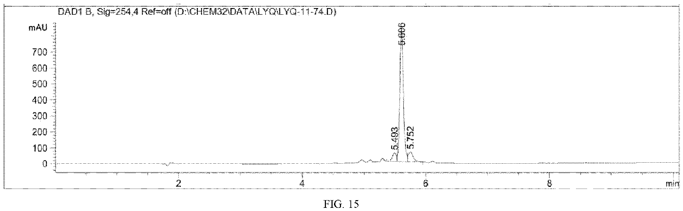

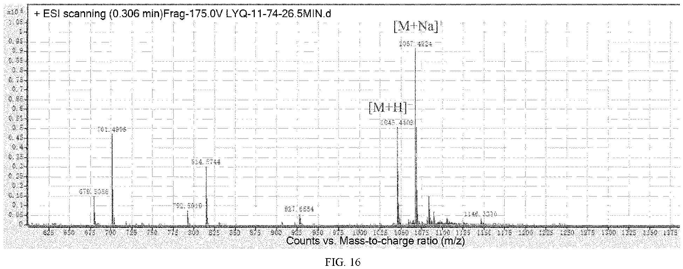

The polypeptide compound **DP-18** was prepared with reference to Example 44, and a separation yield was 56%. HPLC and mass spectrometry spectra of the polypeptide compound DP-18 were shown in FIG. 15 and FIG. 16, respectively. Analytical gradient 10-100% B over 10 min, 254 nm. Calculated Mass [M+H]⁺: 1045.4448, Observed Mass [M+H]⁺: 1045.4408.

### Example 49 Preparation of a polypeptide compound DP-19

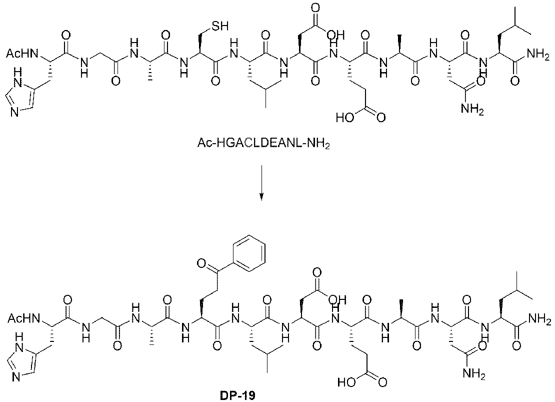

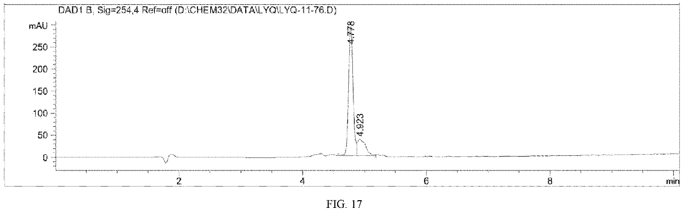

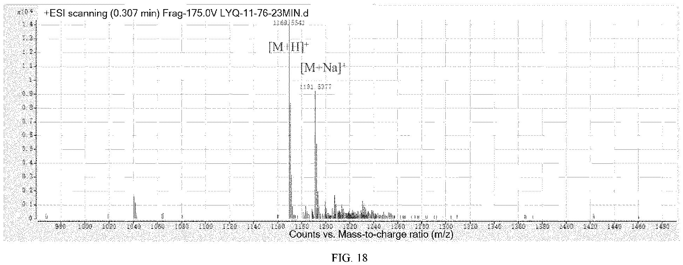

The polypeptide compound **DP-19** was prepared with reference to Example 44, and a separation yield was 61%. HPLC and mass spectrometry spectra of the polypeptide compound DP-19 were shown in FIG. 17 and FIG. 18, respectively. Analytical gradient 10-100% B over 10 min, 254 nm. Calculated Mass [M+H]⁺: 1169.5586, Observed Mass [M+H]⁺: 1169.5541.

### Example 50 Preparation of a polypeptide compound DP-20

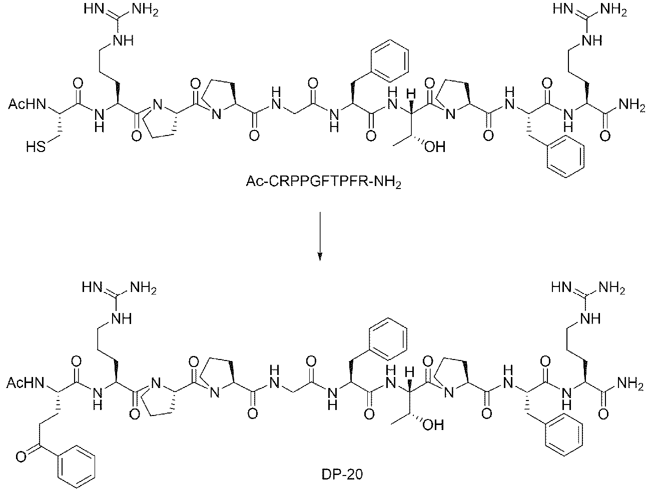

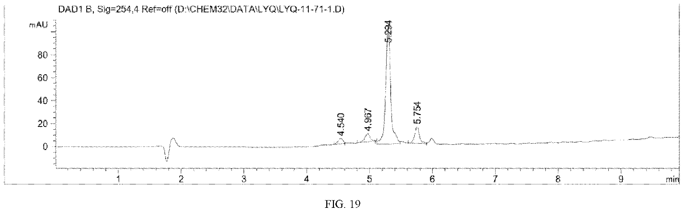

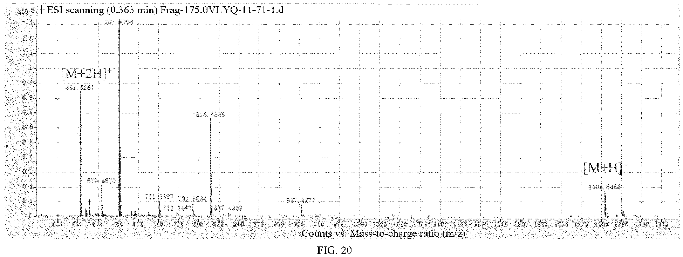

The polypeptide compound **DP-20** was prepared with reference to Example 44, and a separation yield was 65%. HPLC and mass spectrometry spectra of the polypeptide compound DP-20 were shown in FIG. 19 and FIG. 20, respectively. Analytical gradient 10-100% B over 10 min, 254 nm. Calculated Mass [M+H]⁺: 1304.6899, Observed Mass [M+H]⁺: 1304.6466.

This method could also be used for a chemical modification of a protein, as shown in the following examples:

### Example 51 Chemical modification of histone H3

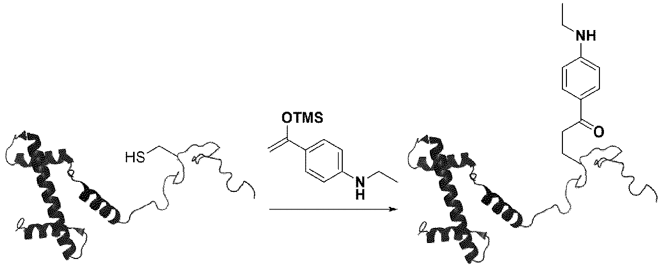

Histone H3 had a molecular weight of 15,214. The histone (100 µL, 66 µM) was added to phosphate-buffered saline (500 mM, pH 8, 3 M Gdn HCl, 90 µL). To a reaction solution, 100 µL of DMSO was added, and then pentafluoropyridine (10 µL, concentration: 66 mM, 100 eq) was added. Stirring was conducted at room temperature to allow a reaction for 30 min. Anhydrous Zn(OAc)₂ (1 µL, concentration: 66 mM, 10 eq), Ir(ppy)₃ (1 µL, concentration: 66 mM, 10 eq), and a silyl enol ether (30 µL, concentration: 220 mM in DMSO, 1,000 eq) were added. Nitrogen replacement was conducted 3 times. Stirring was conducted for 2 h under the irradiation of blue LED.

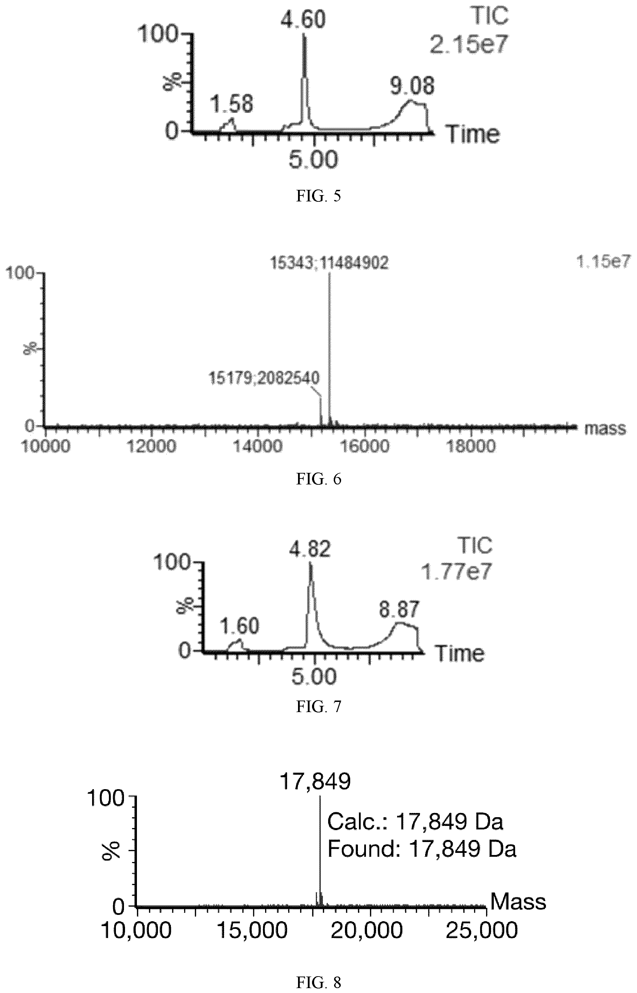

FIG. 5 is an HPLC spectrum of a product from the chemical modification of histone H3. It can be seen from this figure that a reaction conversion rate is very high and a product peak is a main peak.

FIG. 6 is a mass spectrometry spectrum of the product from the chemical modification of histone H3. It can be seen from this figure that an expected protein modification product is obtained, and a molecular weight of the product is in agreement with an expected molecular weight. Calculated mass: 15343 Da; Observed mass: 15343 Da.

### Example 52 Chemical modification of human histone eH3.1

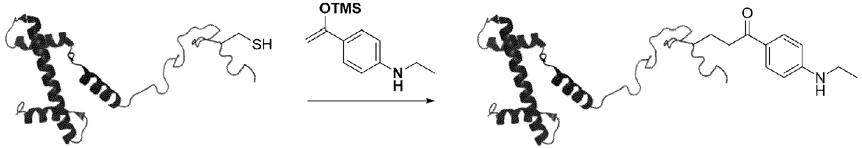

Human histone eH3.1 had a molecular weight of 17,720. The histone eH3.1 (100 µL, 66 µM) was added to phosphate-buffered saline (500 mM, pH 8, 3 M Gdn HCl, 90 µL). To a reaction solution, 100 µL of DMSO was added, and then pentafluoropyridine (10 µL, concentration: 66 mM, 100 eq) was added. Stirring was conducted at room temperature to allow a reaction for 30 min. Anhydrous Zn(OAc)₂ (1 µL, concentration: 66 mM, 10 eq), Ir(ppy)₃ (1 µL, concentration: 66 mM, 10 eq), and a silyl enol ether (30 µL, concentration: 220 mM in DMSO, 1,000 eq) were added. Nitrogen replacement was conducted 3 times. Stirring was conducted for 2 h under the irradiation of blue LED.

FIG. 7 is an HPLC spectrum of a product from the chemical modification of human histone eH3.1. It can be seen from this figure that a reaction conversion rate is very high and a product peak is a main peak.

FIG. 8 is a mass spectrometry spectrum of the product from the chemical modification of human histone eH3.1. It can be seen from this figure that an expected protein modification product is obtained, and a molecular weight of the product is in agreement with an expected molecular weight. Calculated mass: 17849 Da; Observed mass: 17849 Da.

### Example 53 Chemical modification of phosphate transporter PstS-D178C

Phosphate transporter PstS-D178C had a molecular weight of 34,541. The PstS-D178C (100 µL, 66 µM) was added to phosphate-buffered saline (500 mM, pH 8, 3 M Gdn HCl, 90 µL). To a reaction solution, 100 µL of DMSO was added, and then pentafluoropyridine (10 µL, concentration: 66 mM, 100 eq) was added. Stirring was conducted at room temperature to allow a reaction for 30 min. Anhydrous Zn(OAc)₂ (1 µL, concentration: 66 mM, 10 eq), Ir(ppy)₃ (1 µL, concentration: 66 mM, 10 eq), and a silyl enol ether (30 µL, concentration: 220 mM in DMSO, 1,000 eq) were added. Nitrogen replacement was conducted 3 times. Stirring was conducted for 2 h under the irradiation of blue LED.

FIG. 7 is an HPLC spectrum of a product from the chemical modification of PstS-D178C. It can be seen from this figure that a reaction conversion rate is very high and a product peak is a main peak.

FIG. 8 is a mass spectrometry spectrum of the product from the chemical modification of human histone eH3.1. It can be seen from this figure that an expected protein modification product is obtained, and a molecular weight of the product is in agreement with an expected molecular weight. Calculated mass: 17849 Da; Observed mass: 17849 Da.

### Example 54 Plasma stability test

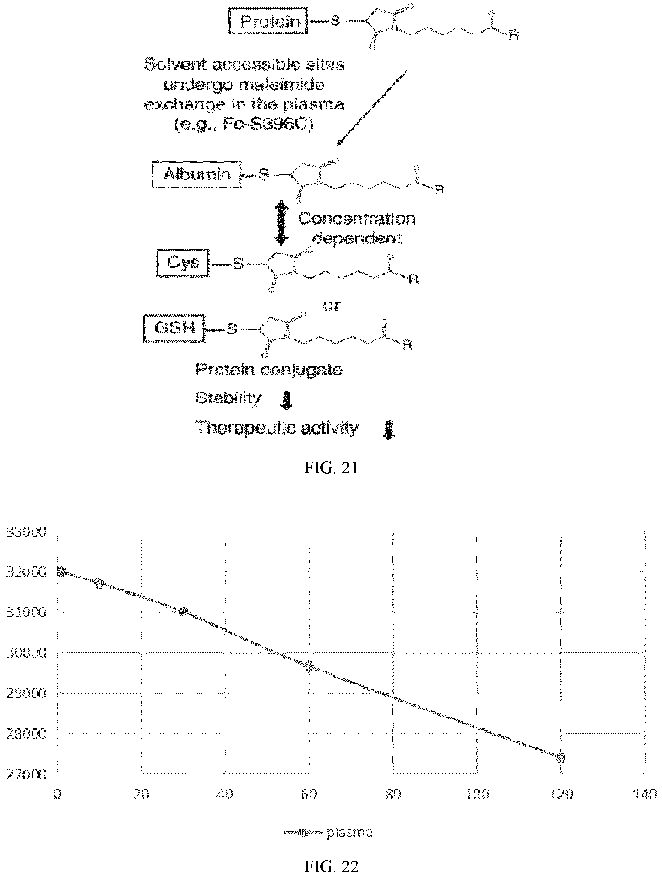

Currently, one of the common linking modes for ADCs is characterized by forming a C-S bond through an addition reaction of sulfhydryl on a cysteine residue in an antibody with a small molecule fragment including maleimide. However, such a linker will undergo an inverse addition reaction in the plasma to produce a small molecule fragment including maleimide. This small molecule fragment will undergo an addition reaction with albumin, glutathione, etc. in the plasma to produce an albumin-small molecule conjugate, a glutathione-small molecule conjugate, etc., which is an important reason for the toxicity and side effects of ADCs (as shown in FIG. 21). This phenomenon has been investigated in detail in the literature (as shown in FIG. 21, which is edited from Nat. Biotechnol 30, 184-189 (2012)).

In the method of the present disclosure, a carbon-carbon bond can be constructed on the basis of cysteine. The carbon-carbon bond is the most stable and is much more stable than the C-S bond. Therefore, the method developed by the present disclosure can be widely used in ADCs, and can eliminate the toxicity caused by the breakage of C-S bonds in ADCs. In order to verify this advantage, based on a synthetic polypeptide fragment, a plasma stability test was conducted in this example.

A method was as follows:
About 3 µL of a 500 µg/mL compound was taken and added to 297 µL of plasma. Incubation was conducted at 37°C for 0 h to 3 h. 20 µL of a sample was collected at 1 min, 10 min, 30 min, 60 min, and 120 min. Then 100 µL of acetonitrile was added to precipitate proteins. Then 1 µL of a sample was collected and tested by liquid chromatography-tandem triple quadrupole mass spectrometer (LC-MS/MS) to determine a downward response of the compound in the plasma. The stability of the compound in the plasma could be quantified through plotting.

The plasma stability of a compound 1 determined by the above method was shown in FIG. 22. It can be seen that 2 h later, about 14% of the compound was degraded. A structure of the compound 1 was shown in the following formula, which was attributed to the instable C-S bond.

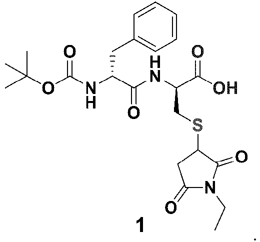

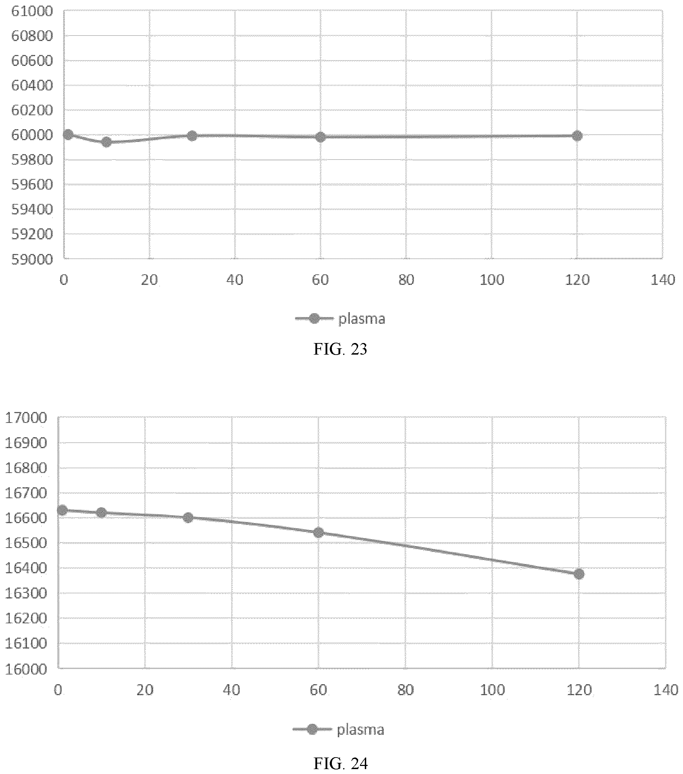

As a contrast, the plasma stability of the compound DP-04 (Example 32) determined by the above method was shown in FIG. 23. It can be seen that 2 h later, the degradation of the compound was not observed within an error range.

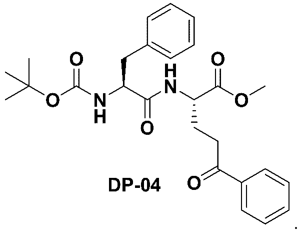

The plasma stability of a compound 2 determined by the above method was shown in FIG. 24. It can be seen that 2 h later, about 1.5% of the compound was degraded.

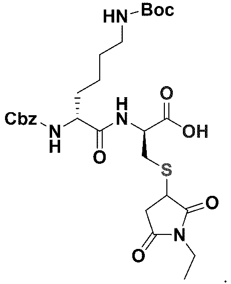

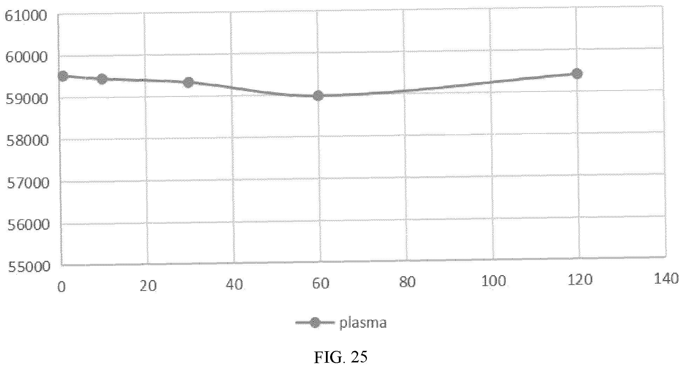

As a contrast, the plasma stability of the compound DP-11 (Example 40) determined by the above method was shown in FIG. 25. It can be seen that 2 h later, the degradation of the compound was not observed within an error range.

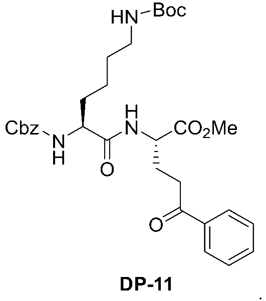

In summary, the present disclosure provides a method for visible light-mediated chemical modification of a polypeptide and protein based on cysteine, including the following steps: a cysteine residue on a cysteine-containing oligopeptide, polypeptide, or protein is allowed to undergo a reaction with a halide to generate a free radical precursor *in situ*; and the free radical precursor is subjected to thiol removal under light induction, and a generated free radical intermediate is allowed to undergo an addition reaction with an olefin or an alkyne, such that sulfhydryl removal and carbon-carbon bond construction are achieved to produce a chemical modification product of the polypeptide and protein. The design strategy of the present disclosure cleverly avoids the influence on a chiral center, and thus the chirality of an amino acid residue can be retained. The whole design is conducive to well avoiding the occurrence of side reactions, and thus the efficiency of the entire conversion is very high. The reactions can be conducted mildly under physiological conditions, and can be suitable for various types of amino acid residues and olefins or alkynes. Therefore, the method has advantages such as high efficiency, wide application range, and maintenance of amino acid residue configurations. The method can be used in the synthesis of unnatural amino acids, and more importantly, can be used in the chemical modification for polypeptides and proteins, including fields such as chemical transformation for proteins, structural modification for enzymes, development of ADCs, and development of degrader-antibody conjugates. Because a carbon-carbon bond has much better stability than a carbon-sulfur bond and can avoid the side effects caused by carbon-sulfur bond breaking, the method has a promising application prospect.

The specific examples of the present disclosure are described above. It should be understood that the present disclosure is not limited to the above specific implementations, and a person skilled in the field can make various variations or modifications within the scope of the claims without affecting the essence of the present disclosure.

## Claims

1. A method for visible light-mediated chemical modification of a polypeptide and protein based on cysteine, comprising the following steps:
S1, allowing a cysteine residue on a cysteine-containing oligopeptide, polypeptide, or protein to undergo a reaction with an activating reagent to generate a free radical precursor *in situ*; and
S2, subjecting the free radical precursor to thiol removal under induction of a photocatalyst, and allowing a generated free radical intermediate to undergo an addition reaction with an olefin or an alkyne, such that sulfhydryl removal and carbon-carbon bond construction are achieved to produce a chemical modification product of the polypeptide and protein.

2. A method for visible light-mediated chemical modification of a polypeptide and protein based on cysteine, comprising the following steps:
S1', allowing a cysteine residue on a cysteine-containing oligopeptide, polypeptide, or protein to undergo a S_{N}Ar reaction with a halogen or halogenoid-substituted aromatic hydrocarbon to generate a free radical precursor *in situ*; and
S2', subjecting the free radical precursor to aryl thiol removal under light induction, and allowing a generated free radical intermediate to undergo an addition reaction with an olefin, such that sulfhydryl removal and carbon-carbon bond construction are achieved to produce a chemical modification product of the polypeptide and protein.

3. The method for chemical modification of the polypeptide and protein based on the cysteine according to claim 1 or 2, wherein the cysteine residue is at a C-terminus and/or an N-terminus of the oligopeptide, polypeptide, or protein and/or in a middle of a peptide chain.

4. The method for chemical modification of the polypeptide and protein based on the cysteine according to claim 1, wherein the activating reagent is a substituted aromatic or heteroaromatic hydrocarbon comprising a leaving group; and the leaving group comprises a halogen and a halogenoid.

5. The method for chemical modification of the polypeptide and protein based on the cysteine according to claim 1, wherein the activating reagent is selected from pyrimidine, 1,3,5-triazine, thiazole, imidazole, oxazole, benzoxazole, benzothiazole, or benzimidazole that is substituted with a leaving group or selected from electron-deficient aromatic ring compounds 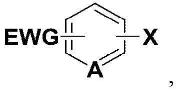 wherein X represents a leaving group and the leaving group comprises a halogen and a halogenoid; A represents a carbon atom or a nitrogen atom; EWG represents an electron-withdrawing group; the halogen is selected from fluorine, chlorine, bromine, and iodine; the halogenoid is selected from cyano, thiocyano, selenium cyano, thioether, a sulfone group, and trifluoromethanesulfonic acid; and the electron-withdrawing group is selected from fluorine, chlorine, bromine, iodine, carbonyl, carboxyl, an ester group, amido, nitro, polyfluoroalkyl, sulfonyl, sulfonamido, a sulfonyl ester group, cyano, and a tertiary amine cation.

6. The method for chemical modification of the polypeptide and protein based on the cysteine according to claim 1 or 5, wherein the activating reagent is selected from one of compounds with the following structural formulas: 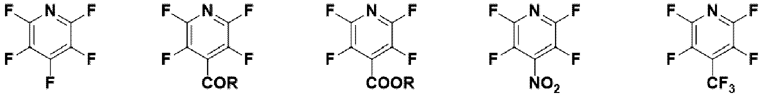 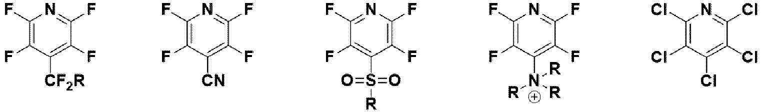 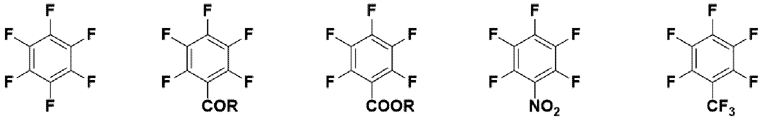 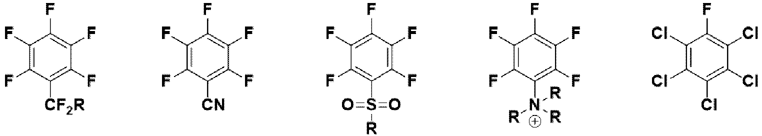 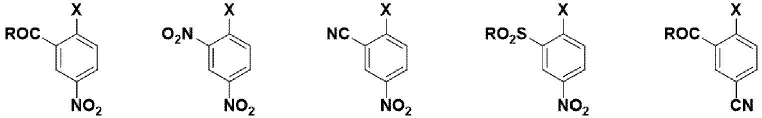 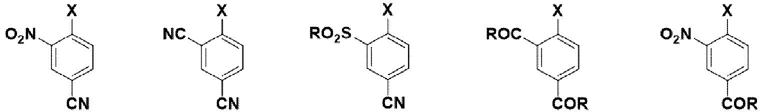 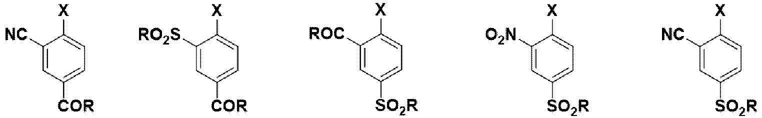 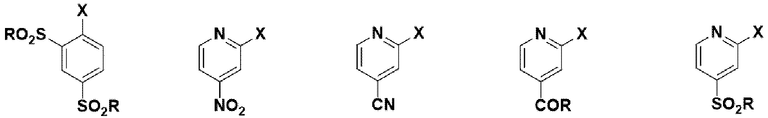 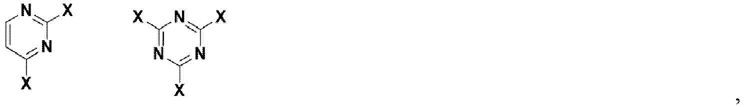 wherein X represents a halogen or a halogenoid; the halogen comprises fluorine, chlorine, bromine, and iodine and the halogenoid comprises cyano, thiocyano, selenium cyano, thioether, a sulfone group, and a trifluoromethanesulfonyl group; and R is alkyl, aryl, or a heterocycle.

7. The method for chemical modification of the polypeptide and protein based on the cysteine according to claim 2 or 4, wherein the halogen or halogenoid-substituted aromatic hydrocarbon is selected from one of compounds with the following structural formulas:

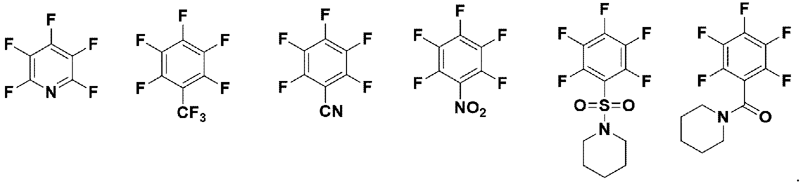

8. The method for chemical modification of the polypeptide and protein based on the cysteine according to claim 1, wherein the activating reagent is a salt and has a structural formula selected from one of the following structural formulas: 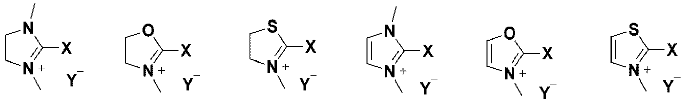 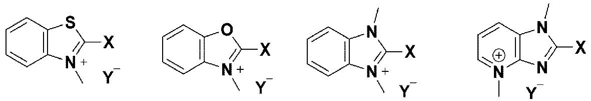 , wherein X represents a leaving group, and comprises one of the following structural formulas: 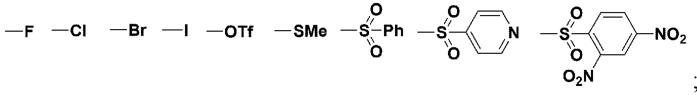 and Y⁻ comprises Cl⁻, Br, I⁻, BF₄⁻, PF₆⁻, or OTF⁻.

9. The method for chemical modification of the polypeptide and protein based on the cysteine according to claim 1, wherein the alkyne is an aryl alkyne or an aliphatic alkyne; and
the olefin is a monosubstituted olefin, a disubstituted olefin, or a trisubstituted olefin and is preferably selected from compounds with the following structural formulas: 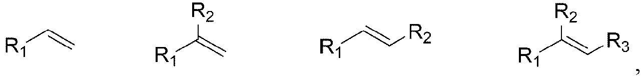 wherein R₁, R₂, and R₃ each are aryl, alkyl, alkenyl, or alkynyl.

10. The method for chemical modification of the polypeptide and protein based on the cysteine according to claim 1 or 2, wherein the olefin is selected from one of compounds with the following structural formulas: 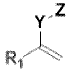
, wherein R₁ is aryl, alkyl, alkenyl, or alkynyl, Y is an oxygen atom or NH, and Z is silyl or acyl; and the silyl comprises at least one of trimethylsilyl (TMS), triethylsilyl, triisopropylsilyl, tert-butyldimethylsilyl, and tert-butyldiphenylsilyl, and the acyl comprises at least one of acetyl, trimethylacetyl, benzoyl, isopropoxycarbonyl, tert-butoxycarbonyl, benzyloxycarbonyl, methanesulfonyl, and p-toluenesulfonyl; 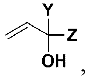
wherein Y and Z each are aryl, alkyl, alkenyl, or alkynyl; 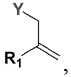
wherein R₁ is aryl, alkyl, alkenyl, or alkynyl and Y is one of Cl, Br, I, a sulfone group, polysubstituted silyl, and a polysubstituted tin group; the polysubstituted silyl comprises TMS, triethylsilyl, triisopropylsilyl, tert-butyldimethylsilyl, and tert-butyldiphenylsilyl; and the polysubstituted tin group comprises a trimethyltin group, a tributyltin group, a triallyltin group, and a triphenyltin group; 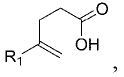
wherein R₁ is aryl, alkyl, alkenyl, or alkynyl; 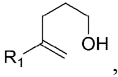
wherein R₁ is aryl, alkyl, alkenyl, or alkynyl; 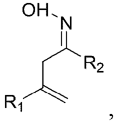
, wherein R₁ and R₂ each are aryl, alkyl, alkenyl, or alkynyl; 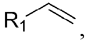
wherein R₁ is aryl or heteroaryl; and 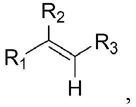
wherein at least one of R₁ and R₂ comprises an electron-withdrawing group, and the electron-withdrawing group is selected from carbonyl, carboxyl, an ester group, amido, nitro, polyfluoroalkyl, sulfonyl, sulfonamido, a sulfonyl ester group, and cyano; and R₃ is aryl, alkyl, alkenyl, or alkynyl.

11. The method for chemical modification of the polypeptide and protein based on the cysteine according to claim 10, wherein a functional group or a macromolecular fragment is introduced through at least one of the R₁, R₂, Y, and Z groups; and the functional group comprises one or more of hydroxyl, an ether bond, alkenyl, alkynyl, azido, sulfo, amino, halogen, carboxyl, an ester group, and amido, and the macromolecular fragment comprises one or more of an unnatural amino acid group, a fluorophore group, a drug molecule group, SiRNA, an antibody, and a ligand binding to an E3 ubiquitin ligase.

12. The method for chemical modification of the polypeptide and protein based on the cysteine according to claim 10, wherein the alkyl is linear alkyl or branched alkyl or is linear alkyl or branched alkyl with a heteroatom substitution in a middle or at an end, and the heteroatom substitution comprises a substitution with one or more of oxygen, nitrogen, sulfur, fluorine, chlorine, bromine, iodine, boron, silicon, and phosphorus;
the aryl is carbocyclic or heterocyclic aryl, or is carbocyclic or heterocyclic aryl with substituents, wherein the substituents are located at any position of a ring and could be monosubstituted or polysubstituted; the aryl comprises, but is not limited to, phenyl, naphthyl, pyridyl, thienyl, indolyl, and quinolyl; and the substituent comprises, but is not limited to, alkyl, alkenyl, alkynyl, halogen, nitro, an ester group, amido, amino, hydroxyl, sulfonamido, an ether group, or a thioether group;
the alkenyl is linear or branched alkenyl or is linear or branched alkenyl with a heteroatom substitution in a middle or at an end, and the heteroatom substitution comprises oxygen, nitrogen, sulfur, fluorine, chlorine, bromine, iodine, boron, silicon, and phosphorus substitutions;
the alkynyl is an alkyne or is an alkyne with a heteroatom substitution in a middle or at an end, and the heteroatom substitution comprises a substitution with one or more of oxygen, nitrogen, sulfur, fluorine, chlorine, bromine, iodine, boron, silicon, and phosphorus.

13. The method for chemical modification of the polypeptide and protein based on the cysteine according to claim 1, wherein the olefin is a silyl enol ether olefin; and the olefin is selected from compounds with the following structural formulas: 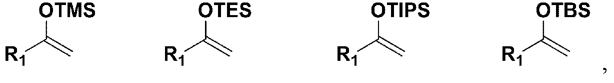 wherein R₁ is alkyl and aryl with or without a substituent; during the addition reaction, a functional group or a macromolecular fragment is introduced through the R₁ group; and the functional group comprises one or more of hydroxyl, a carboxyl-ether bond, alkenyl, alkynyl, azido, alkynyl, sulfo, amino, halogen, carboxyl, and an ester group, and the macromolecular fragment comprises one or more of an unnatural amino acid group, a fluorophore group, a drug molecule group, SiRNA, an antibody, and a ligand binding to an E3 ubiquitin ligase.

14. The method for chemical modification of the polypeptide and protein based on the cysteine according to claim 1, wherein the olefin is an enol ester, enamine, or terminal olefin; and the alkyne is an aryl alkyne or an aliphatic alkyne.

15. The method for chemical modification of the polypeptide and protein based on the cysteine according to claim 1 or 2, wherein the olefin is selected from one of compounds with the following structural formulas: 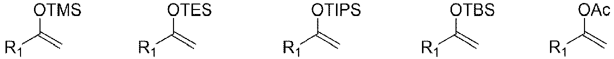 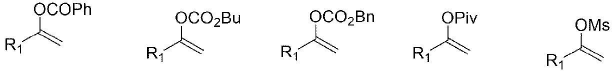 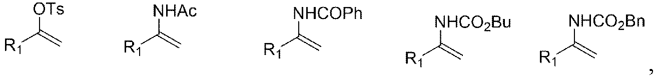 wherein R₁ comprises aryl, alkyl, alkenyl, or alkynyl; during the addition reaction, a functional group or a macromolecular fragment is introduced through the R₁ group; and the functional group comprises one or more of hydroxyl, an ether bond, alkenyl, alkynyl, azido, sulfo, amino, halogen, carboxyl, an ester group, and amido, and the macromolecular fragment comprises one or more of an unnatural amino acid group, a fluorophore group, a drug molecule group, RNA, an antibody, and a ligand binding to an E3 ubiquitin ligase.

16. The method for chemical modification of the polypeptide and protein based on the cysteine according to claim 1 or 2, wherein the olefin has one of the following structural formulas, wherein linker represents a small-molecule organic compound fragment for linking, azide represents azido, alkyne represents alkynyl, flurophore represents a fluorophore group, drug represents a drug molecule group, E3 ligase ligand represents a ligand binding to an E3 ubiquitin ligase, and RNA represents an RNA nucleotide:

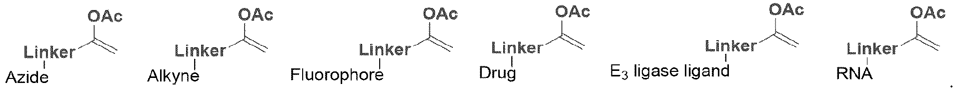

17. The method for chemical modification of the polypeptide and protein based on the cysteine according to claim 1 or 2, wherein the olefin or the alkyne is selected from one of compounds with the following structural formulas, wherein drug represents monomethyl auristatin E (MMAE), monomethyl auristatin F (MMAF), calicheamicin, duocarmycin, a mertansine derivative DM1, a topoisomerase I inhibitor SN-38, a camptothecin (CPT) analogue DXd, or pyrrolobenzodiazepine (PBD):

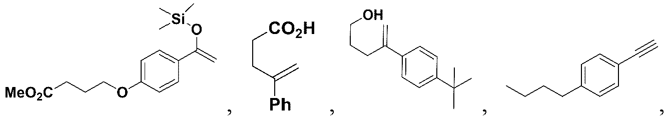

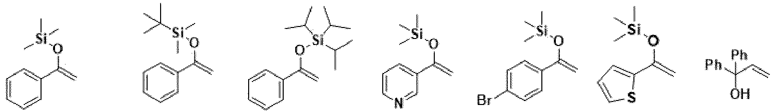

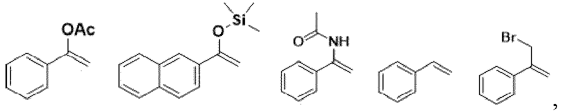

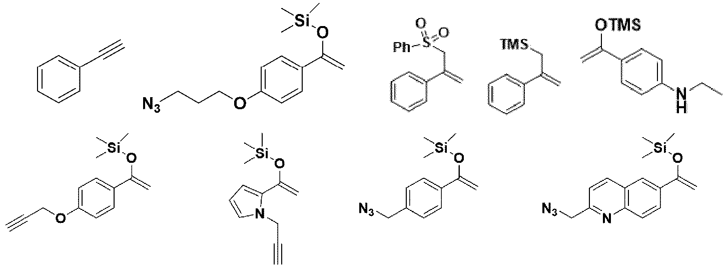

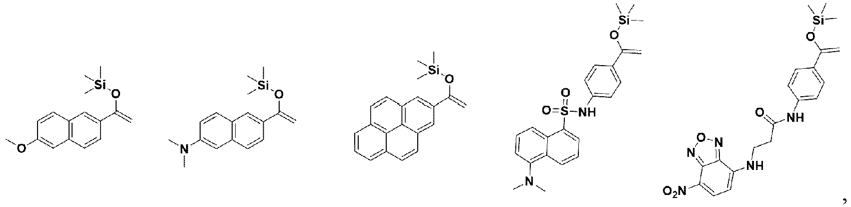

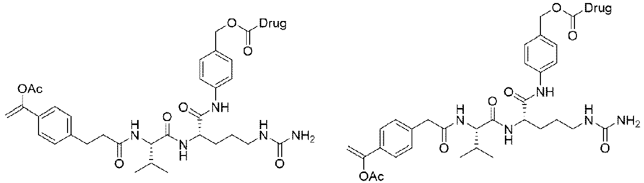

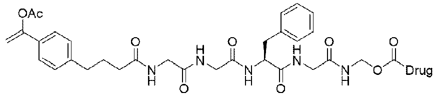

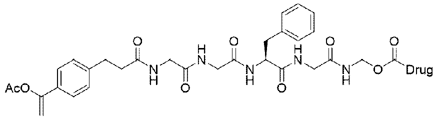

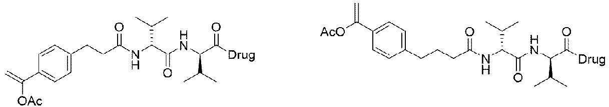

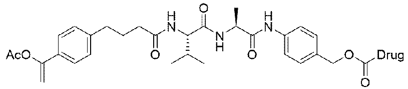

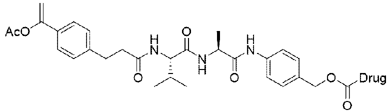

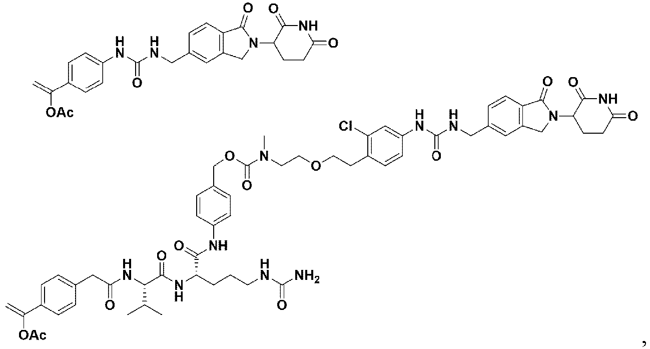

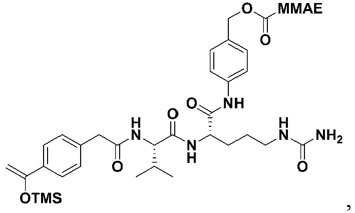

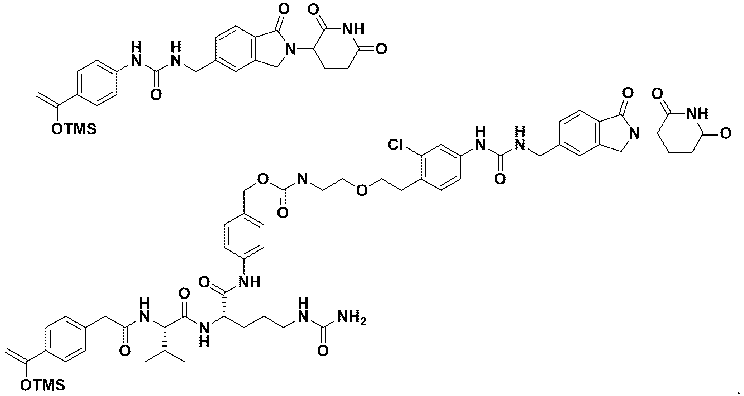

18. The method for chemical modification of the polypeptide and protein based on the cysteine according to claim 1, wherein in the S2, during the addition reaction with the olefin or the alkyne, a plurality of additives are added; and the plurality of additives comprise Hantzsch ester, water, TMS cyanide (TMSCN), an azide, hexamethyldisilane, dihydropyridine, triethylsilane, and thiophenol.

19. The method for chemical modification of the polypeptide and protein based on the cysteine according to claim 1 or 2, wherein in the S1, the reaction is conducted in a presence of an alkali and a solvent; the alkali is selected from N,N-diisopropylethylamine (DIPEA), sodium bicarbonate, sodium carbonate, zinc acetate, potassium acetate, or a weakly-alkaline buffer solution; the solvent is selected from one of an organic solvent, water, a buffer solution, a mixed solvent of the organic solvent and the water, and a mixed solvent of the organic solvent and the buffer solution; and the organic solvent is selected from dimethyl sulfoxide (DMSO), acetonitrile, N,N-dimethylformamide (DMF), tetrahydrofuran (THF), acetone, and ethyl acetate.

20. The method for chemical modification of the polypeptide and protein based on the cysteine according to claim 1 or 2, wherein in the S2, the reaction is conducted in a presence of the photocatalyst and light.

21. The method for chemical modification of the polypeptide and protein based on the cysteine according to claim 1 or 2, wherein in the S2, the reaction is conducted in a presence of the photocatalyst and blue light; during the reaction, an inorganic salt is added, and an electron donor-acceptor (EDA) complex is generated to stimulate production of a free radical; the photocatalyst is an Ir photocatalyst or a Ru photocatalyst, and is preferably Ir(ppy)₃, Ru(bpm)₃Cl₂, Ru(bpy)₃Cl₂, [Ir(spppy)₃]³⁻, [Ir(s^{F}ppy)₃]⁻, [Ir(s^{dF}ppy)₃]³⁻, or [Ir(s^{CH2}ppy)₃]⁻; and the inorganic salt is an inorganic alkali or an inorganic acid, and is preferably at least one of zinc acetate, zinc trifluoroacetate, sodium bicarbonate, sodium carbonate, potassium carbonate, copper acetate, sodium acetate, potassium acetate, cesium carbonate, triphenylphosphine, zinc chloride, and zinc fluoride.

22. A use of the method for chemical modification of the polypeptide and protein based on the cysteine according to any one of claims 1 to 21 in structural modification for the polypeptide and protein, labeling and tracing for a protein, a development of an antibody-drug conjugate (ADC), a development of a degrader-antibody conjugate, a development of an antibodyoligonucleotide conjugate (AOC), a development of an antibody-cell conjugate (ACC), or a development of an antibody-photosensitizer conjugate.
